# FASCICULE DE BREVET EUROPEEN

(11) **EP 3 226 923 B1**
(45) Date de publication et mention de la délivrance du brevet: **17.02.2021**
(21) Numéro de dépôt: 15817457.3
(22) Date de dépôt: 01.12.2015
(51) Int. Cl.: A61L 27/20, A61L 27/36, A61L 27/38, C08L 5/08

(54) **GEL DE CARTILAGE POUR LA RÉPARATION CARTILAGINEUSE, COMPRENANT DU CHITOSANE ET DES CHONDROCYTES**
KNORPELGEL ZUR KNORPELREPARATUR MIT CHITOSAN UND CHONDROZYTEN
CARTILAGE GEL FOR CARTILAGE REPAIR, COMPRISING CHITOSAN AND CHONDROCYTES

(30) Priorité: 01.12.2014 FR 1461746
(43) Date de publication de la demande: 11.10.2017
(73) Titulaire: Advanced Chitosan Solutions Biotech, 69100 Villeurbanne (FR); Centre National de la Recherche Scientifique, 75794 Paris Cedex 16 (FR); Université Claude Bernard Lyon 1, 69622 Villeurbanne, Cedex (FR)
(72) Inventeur: HAZOT, Pascale, 69100 Villeurbanne (FR); MALLEIN GERIN, Frédéric, 69007 Lyon (FR)
(74) Mandataire: Ernest Gutmann - Yves Plasseraud S.A.S.
(86) Numéro de dépôt international: PCT/FR2015/053271
(87) Numéro de publication internationale: WO 2016/087762

(56) Documents cités:
- WO-A1-2011/104131
- US-A1- 2009 022 770
- CLARA R. CORREIA ET AL: "Chitosan Scaffolds Containing Hyaluronic Acid for Cartilage Tissue Engineering", TISSUE ENGINEERING PART C: METHODS, vol. 17, no. 7, 1 juillet 2011 (2011-07-01), pages 717-730, XP055211544, ISSN: 1937-3384, DOI: 10.1089/ten.tec.2010.0467

## Description

La présente invention a trait à des compositions permettant notamment la reconstruction du cartilage, et à un procédé permettant d'obtenir de telles compositions. Plus particulièrement, la présente demande concerne un environnement ou structure extrêmement favorable non seulement à la prolifération de cellules aptes à former du cartilage hyalin, mais également à la synthèse de matrice extracellulaire de cartilage par ces cellules; les cellules dans cet environnement ou structure constituent une composition implantable qui peut être greffée au niveau des lésions de cartilage chez l'homme ou chez l'animal. La structure constitue par ailleurs également un environnement favorable lors de l'implantation.

Le cartilage, ou tissu cartilagineux, est constitué de cellules spécifiques, à savoir de chondrocytes, réparties au sein d'une matrice extracellulaire, comportant au moins 80% d'eau. Les chondrocytes sont capables de synthétiser ou dégrader les composants de la matrice extracellulaire du cartilage, composée de glycosaminoglycanes et de fibres de collagène, essentiellement de type II pour le cartilage hyalin. Les chondrocytes sont donc responsables non seulement de la synthèse mais aussi du maintien du tissu cartilagineux.

Le cartilage, et particulièrement le cartilage articulaire à l'âge adulte, possède une très faible capacité d'autoréparation, du fait notamment de son caractère avasculaire et du fait que les chondrocytes matures ne prolifèrent pas. Les lésions cartilagineuses sont donc essentiellement irréversibles et constituent par conséquent une cause importante de douleur et de handicap, notamment suite à un traumatisme, à l'usure mécanique ou à une maladie dégénérative articulaire comme l'arthrose. A l'heure actuelle, il n'existe pas de solution thérapeutique entièrement satisfaisante, permettant de traiter les lésions cartilagineuses, et tout particulièrement les lésions larges.

Différents procédés chirurgicaux ont été testés, consistant soit à combler la lésion avec des matériaux visant à mimer les propriétés élastiques et compressibles du cartilage naturel, soit à greffer des cellules, notamment des chondrocytes, dans l'espoir qu'elles synthétisent *de novo* de la matrice extracellulaire et réparent ainsi la lésion. Une approche plus prometteuse repose sur la greffe d'un tissu cartilagineux, c'est-à-dire d'un néo-tissu obtenu à partir de chondrocytes. La greffe de chondrocytes autologues (« ACI » pour autologous chondrocytes implantation) est utilisée depuis plusieurs décennies maintenant pour traiter des patients atteints de lésions cartilagineuses. Cependant, une difficulté majeure dans la greffe de chondrocytes, et tout particulièrement de chondrocytes autologues, réside dans le nombre de cellules à implanter. En effet, il ne peut généralement être prélevé qu'un nombre réduit de chondrocytes à partir de cartilage natif du patient qui doit être traité ; il est par conséquent nécessaire de procéder à une première étape d'expansion des chondrocytes prélevés afin d'augmenter drastiquement leur nombre. Or l'expansion *in vitro* de chondrocytes jusqu'à l'obtention d'un nombre suffisant de cellules en vue d'une implantation s'avère très délicate. Durant cette expansion, il est en effet connu que les chondrocytes se dédifférencient et perdent leur phénotype chondrocytaire, exprimant alors du collagène de type I plutôt que du collagène de type II, si bien qu'une fois réimplantés, ils ne conduisent pas à la formation d'un tissu aux propriétés satisfaisantes, mais plutôt à un tissu de style cicatriciel ou bien, dans le cadre d'une réparation cartilagineuse, à la formation d'un fibrocartilage non-fonctionnel, essentiellement composé de collagène de type I. Certains auteurs ont proposé des milieux de culture permettant de redifférencier les chondrocytes qui se sont dédifférenciés, lors de l'étape de prolifération en monocouches. Cependant ces procédés nécessitent des temps relativement longs, de l'ordre de 4 semaines pour l'étape de prolifération (Liu et al, 2007) au cours de laquelle les chondrocytes doivent subir plusieurs passages en vue d'atteindre un nombre suffisant. Or il est constaté que la re-différenciation est plus compliquée après notamment 2 passages (Hautier et al, 2008).

D'autres ont testé la re-différenciation des chondrocytes, après l'étape de prolifération en monocouches, par ensemencement dans des structures 3D (tridimensionnelles), dites 'scaffolds'. Ces structures offrent l'avantage de mimer l'architecture du tissu cartilagineux. Un tel environnement est favorable à la re-différenciation des chondrocytes et à la synthèse de matrice extracellulaire comme constaté par la sécrétion de proteoglycanes et de collagène de type II (Tallheden et al, 2005). Toutefois, l'étape de multiplication préalable est volontairement écourtée en vue de limiter la dédifférenciation des chondrocytes.
L'ensemencement en structure 3D peut également offrir l'avantage de faciliter l'implantation in *vivo* des chondrocytes, de limiter les fuites cellulaires et de traiter des lésions larges.
De nombreux matériaux ont été testés pour l'ensemencement de chondrocytes en structure 3D ; on peut citer l'alginate, le collagène, le polyéthylène glycol et l'acide polylactique. Les plus appropriés sont ceux qui répondent aux propriétés biologiques suivantes: cytocompatibilité, faible immunogénéicité, et biodégradabilité avec des produits de dégradation non toxiques. Ainsi les polymères naturels, qui présentent ces propriétés biologiques, certainement de par leurs similarités chimiques et biologiques avec les tissus vivants, se présentent comme des candidats de choix. Les polymères naturels sont fortement biocompatibles, aisément biorésorbables et bioassimilables par l'organisme.

Un polymère naturel souvent cité pour ses propriétés attrayantes comme biomatériau de base de structures tridimensionnelles, est le chitosane.
En effet, d'après la littérature, ce biopolymère est connu pour être biodégradable, biocompatible, non-toxique, hémocompatible, cytocompatible, et en plus, bioactif, hémostatique, cicatrisant, bactériostatique et fongistatique.
De plus, le chitosane est connu pour favoriser l'adhésion cellulaire. Il est également apprécié pour sa capacité à maintenir le phénotype chondrocytaire et stimuler le processus de synthèse de matrice extracellulaire lors de la culture de chondrocytes. Par ailleurs, il n'est rapporté aucune infection ou allergie liée au chitosane, il est donc non immunogène. Quant à son temps de résorption, il est possible de le faire varier en jouant sur ses propriétés physico-chimiques.
Le chitosane est d'une d'utilisation relativement facile et il peut être produit sous différentes formes physiques, notamment des solutions, des films (Lahiji, et al, 2000), des fibres, des éponges, des billes, des hydrogels ou des microparticules. Il a de ce fait été utilisé dans des structures extrêmement variées.
Dans le cadre de l'ensemencement de chondrocytes en structure 3D, une attention particulière est portée aux structures permettant à la fois une bonne distribution et un bon maintien des chondrocytes ainsi qu'un environnement favorable à la stimulation de leur potentiel chondrogénique. A cet effet, différentes approches ont été envisagées. Parmi les différentes structures testées, on peut citer les billes ou fragments de matériau, les polymères visant à encapsuler les cellules et les hydrogels dont la taille des pores est ajustée afin que les chondrocytes puissent s'y loger. Certains auteurs ont envisagé des compositions injectables, avec un polymère encapsulant les chondrocytes (WO2011/104131, Univ. de Liège et al). Selon certaines variantes, le polymère est réticulé in situ, une fois injecté avec les chondrocytes au niveau de la lésion (Hoemann et al, 2005). Cette approche a toutefois l'inconvénient que les chondrocytes injectés ont tendance à ne pas rester sur le site d'injection, du fait du temps de polymérisation. A l'inverse, d'autres auteurs ont testé des compositions, à partir d'un polymère réticulé in-situ et permettant la réalisation d'un tissu pouvant être greffé sous forme de plug (Hao et al, 2010). Ces procédés peuvent néanmoins présenter des difficultés lors de l'implantation. Afin de mimer au mieux la structure du tissu cartilagineux, certains auteurs ont testé la forme hydrogel et plus particulièrement l'hydrogel physique, synthétisé sans ajout d'agent de réticulation et favorisant ainsi la biocompatibilité et la biorésorabilité de la structure.
Il a donc été envisagé l'élaboration d'un néo-tissu par la mise en contact avec un hydrogel physique de chitosane (Montembault et al). Cependant, l'étape de multiplication préalable en monocouches est volontairement rapide afin d'éviter ou de limiter la dédifférenciation des chondrocytes avant ensemencement, impliquant par conséquent, une multiplication des cellules relativement faible.
Selon une variante, la structure tridimensionnelle se résorbe en grande partie au cours de l'étape in vitro de synthèse de la matrice extracellulaire, ladite structure ne fait plus partie de la greffe (voir notamment WO02078760, Laboratoires Genevrier et al). Un tel procédé est particulièrement long à mettre en œuvre, de 4 à 6 semaines, avant d'obtenir un néo-tissu apte à être greffé, et ledit néo-tissu n'est plus soutenu par la structure 3D au moment de l'implantation.

Un autre polymère qui a été fréquemment testé dans le domaine de l'implantation de chondrocytes est l'acide hyaluronique. C'est un polysaccharide naturel, également biocompatible et biodégradable. Par ailleurs, il s'agit d'un composant majoritaire du liquide synovial et des glycosaminoglycanes (GAG) présents dans le cartilage articulaire. L'acide hyaluronique aide à protéger les articulations en augmentant la viscosité du liquide synovial et en rendant le cartilage plus élastique. Il a par ailleurs été démontré que l'acide hyaluronique favorise l'expression du phénotype chondrocytaire.
De ce fait, l'acide hyaluronique a parfois été combiné à des compositions de chitosane, dans la culture de chondrocytes.
Des auteurs (Correia et al, 2011) ont proposé par exemple des structures tridimensionnelles de types éponges d'un mélange de chitosane et d'acide hyaluronique pour l'ensemencement de chondrocytes. Cependant ces structures ne permettent pas une répartition homogène des cellules avec l'observation d'un gradient cellulaire croissant de l'extérieur vers l'intérieur de la structure.
Le document de Denuzière et al (1998) décrit des éponges de complexes polyélectrolytes notamment à base de chitosane et d'acide hyaluronique. Les chaines polycationiques du chitosane interagissent électrostatiquement avec les chaines polyanioniques de l'acide hyaluronique. La conclusion qui s'en dégage est cependant que les éponges de chitosane seul sont à préférer, par rapport à des éponges de chitosane complexés à des GAG tels que l'acide hyaluronique, pour la prolifération des chondrocytes et pour d'autres propriétés biologiques associées.
Il a également été proposé un hydrogel de chitosane et d'acide hyaluronique photo-réticulé au sein duquel des chondrocytes sont encapsulés (Park et al, 2013). Cependant, une telle encapsulation ne favorise pas suffisamment la prolifération malgré des temps de culture relativement longs (facteur de prolifération 4 en 3 semaines).
Par ailleurs, bien que le chitosane semble posséder, d'après la littérature, des propriétés intéressantes en vue de la reconstruction du cartilage, il est à noter que le taux de prolifération et de synthèse de matrice extracellulaire de chondrocytes sur des éponges de chitosane seul dépend fortement de la taille des pores de la structure (Griffon et al, 2006).
Certains auteurs ont même considéré que le chitosane n'était pas adapté à la prolifération des chondrocytes (Suh et al, 2000).

A l'heure actuelle, aucun de ces environnements 3-D (tridimensionnels) ne résout le problème de la prolifération des chondrocytes, dans un état compatible avec la régénération du tissu cartilagineux en vue d'une greffe. Il existe donc un important besoin d'obtenir, selon un procédé rapide, un nombre satisfaisant de chondrocytes, qui soient différenciés et aptes à générer un tissu cartilagineux avec des propriétés adéquates.
Par ailleurs, l'ensemble des procédés proposent des changements d'environnement entre l'étape de multiplication et l'étape de différenciation ce qui implique des pertes de matière et de temps ainsi que le recours à la trypsination, néfaste pour les cellules.

Les présents inventeurs ont mis au point un procédé permettant d'obtenir des chondrocytes en nombre suffisant, au sein d'une structure assurant à la fois leur parfaite multiplication, leur re-différenciation et la production de matrice cartilagineuse. Cette structure est également appropriée à l'implantation au niveau de la lésion.
Dans ce contexte, les présents inventeurs ont trouvé, de manière parfaitement inattendue, que le chitosane, sous forme de particules d'hydrogel physique, est un excellent environnement pour les chondrocytes, non seulement en vue de la synthèse de matrice extracellulaire de cartilage hyalin, mais également en vue de leur multiplication. En effet, contre toute attente, les inventeurs ont pu réaliser dans la même structure de chitosane, une étape de prolifération permettant d'obtenir un taux de prolifération similaire voire supérieur à celui observé par la technique ordinaire des monocouches (sur plastique ou dans des éponges) et autorisant aisément ensuite la re-différenciation des cellules. Les inventeurs ont par conséquent mis au point un procédé, permettant successivement de multiplier des chondrocytes, directement après extraction à l'état primaire, puis d'induire la re-différenciation et la synthèse de matrice extracellulaire spécifique, au sein d'une même structure, évitant ainsi les étapes de trypsination et de changement de structure. De plus, cette structure est compatible avec l'implantation in vivo sans nécessiter de modification supplémentaire.
Grâce au procédé de l'invention, il est ainsi possible d'obtenir une composition comprenant des chondrocytes, répartis de façon homogène au sein de la structure et avec une densité permettant leur réimplantation, en un temps réduit par rapport à ce qui est décrit jusqu'à ce jour, dans des conditions très favorables à la réparation du tissu cartilagineux, dans une structure directement compatible avec l'implantation.

Dans le cadre de cette description, les termes qui suivent ont plus particulièrement la signification suivante :
Par **chitosane,** on entend un polysaccharide composé d'unités D-glucosamine (unité désacétylée) et de N-acétyl-D-glucosamine (unité acétylée) liées en β-(1-4). Il peut être produit par désacétylation chimique ou enzymatique de la chitine ; il existe également à l'état naturel. Dans le chitosane d'origine naturelle, le polysaccharide est généralement associé à des quantités négligeables de béta-glucan. Dans le cadre de la présente invention, la présence naturelle de beta-glucan, dans des quantités conformes à celles trouvées naturellement dans le chitosane, est sans influence. Un chitosane sera dit « pur » même en cas de présence de beta-glucan, tant que la présence de ce dernier reste inférieure à 5% en masse, par rapport au polysaccharide.
Par **dérivé du chitosane,** on désigne tout polymère de chitosane ayant subi une réaction visant à modifier les groupements chimiques du chitosane pour en changer les fonctionnalités, par exemple méthylation, halogénation .... De préférence, un dérivé de chitosane ne possède pas plus de trois types de dérivation, de préférence uniquement deux types de dérivation, et de préférence encore uniquement un type de dérivation. Des dérivés de chitosane particulièrement envisagés dans le cadre de la présente invention sont le glycol-chitosane, le N-succinyl chitosane, le N- ou le O-Carboxymethyl Chitosane, sans que cette liste ne soit limitative.
Le **degré d'acétylation (DA)** est le pourcentage d'unités acétylées par rapport au nombre d'unités totales (unités acétylées et désacétylées), il peut être déterminé par spectrométrie infrarouge à transformée de Fourier (IRTF) ou par RMN du proton.
Par **hydrogel de chitosane,** on entend que le constituant très majoritaire, donc à plus de 80% voire à plus de 90%, voire 95%, de l'hydrogel (en masse) est le chitosane, abstraction faite de l'eau. De manière similaire, un **hydrogel de dérivé de chitosane** s'entend d'un hydrogel dont le constituant très majoritaire, à plus de 80%, voire plus de 90% ou 95% de l'hydrogel est le dérivé de chitosane, abstraction faite de l'eau.
Un hydrogel de chitosane ou de dérivé de chitosane, s'entend d'un hydrogel comprenant de préférence au moins 70% d'eau, voire au moins 80% d'eau.
Par **hydrogel physique,** on entend un hydrogel obtenu par un procédé de gélification en milieu aqueux ou en milieu hydroalcoolique, ne nécessitant pas l'addition d'agent de réticulation.
Par **phénotype chondrocytaire,** on désigne des cellules de type chondrocytes, exprimant préférentiellement du collagène de type II par rapport au collagène de type I.
Par **acide hyaluronique,** on entend un polysaccharide composé d'unités d'acide D-glucuronique et de D-N-acétylglucosamine, liés entre eux par des liaisons glycosidiques.
Par **dérivé de l'acide hyaluronique,** on désigne tout polymère d'acide hyaluronique ayant subi une réaction visant à modifier les groupements chimiques de l'acide hyaluronique pour en changer les fonctionnalités, par exemple par estérification.

La présente invention concerne donc, selon un premier aspect un procédé in vitro d'obtention d'une composition, qui soit implantable par arthroscopie pour la réparation cartilagineuse, comprenant des particules ou fragments d'hydrogel physique de chitosane ou d'un dérivé de chitosane, et des cellules formant du cartilage, de préférence du cartilage hyalin. La composition obtenue à l'issue de la mise en œuvre du procédé peut être qualifiée de gel de cartilage. Un tel gel de cartilage comprend en effet des cellules, synthétisant de la matrice cartilagineuse, le tout réparti de façon homogène sans gradient cellulaire, dans une structure tridimensionnelle à base de particules de chitosane.

Un tel procédé selon l'invention comprend notamment une étape d'amplification de cellules primaires, dans une structure tridimensionnelle (structure 3D) puis une étape de re-différenciation et d'induction de la synthèse de matrice extracellulaire, au sein de cette même structure tridimensionnelle, c'est-à-dire sans changer les cellules d'environnement.
Comme indiqué plus haut, le chitosane est en effet un biomatériau biocompatible, biorésorbable avec des produits de dégradation non-toxiques, non immunogène, cytocompatible et bioactif. Il est de plus entièrement compatible avec les exigences pharmaceutiques en tant que dispositif implantable. Dans le cadre de l'invention, l'hydrogel de chitosane ou de dérivé de chitosane est un hydrogel physique, obtenu sans ajout d'agent de réticulation.

Par ce procédé, il est possible d'obtenir une composition ou néo-tissu comprenant des chondrocytes, au sein d'une structure matricielle, qui soit immédiatement prête à l'implantation, avec des chondrocytes non endommagés aptes à poursuivre la synthèse de matrice cartilagineuse après réimplantation. Du fait de l'absence de changement de structure 3D, le procédé est plus facile à mettre en œuvre et permet d'obtenir un néo-tissu de bonne qualité. Ce procédé est également plus rapide que ceux décrits dans l'art antérieur.

Le procédé selon l'invention se caractérise donc par les étapes suivantes :
(i.) Amplification de cellules primaires, dans une structure tridimensionnelle comprenant des particules d'hydrogel physique de chitosane pur ou de dérivé de chitosane, puis
(ii.) Induction de la différenciation, ou re-différenciation, et de la synthèse de matrice extracellulaire par lesdites cellules amplifiées, au sein de la structure tridimensionnelle de l'étape (i.).
Ces deux étapes se déroulent successivement et non simultanément en vue d'optimiser les conditions et les rendements de chacune de ces étapes.

### Les cellules :

Les cellules qui sont amplifiées, à la première étape, sont ensemencées dans cette structure 3D. Elles peuvent être soit des chondrocytes, soit des cellules précurseurs de chondrocytes obtenus à partir de cellules souches, par exemple des cellules souches mésenchymateuses, soit des cellules pluripotentes induites (IPS). Il peut s'agir de toutes cellules différenciées en chondrocytes. De façon préférée, il s'agit de chondrocytes, et plus préférentiellement de chondrocytes articulaires.
Dans le cadre de l'invention, il est également possible d'utiliser des co-cultures de chondrocytes différenciés et/ou de cellules souches différenciés en chondrocytes.
Les chondrocytes ou les cellules souches peuvent être obtenus par toute méthode connue de l'homme du métier permettant de récupérer ces cellules à partir d'un échantillon biologique susceptible de les contenir. De telles méthodes sont notamment décrites dans le document FR2965278 (Univ. de Caen Basse-Normandie, et al).
Il s'agit de préférence de cellules humaines ou animales, notamment équines ou canines. Il peut s'agir de cellules articulaires, auriculaires ou bien encore issues de la cloison nasale.
Des cellules particulièrement préférées sont des cellules humaines, par exemple des chondrocytes humains, et plus particulièrement des chondrocytes articulaires humains pour un patient humain. Il en est de même pour un animal, notamment pour un cheval ou un chien.
Les cellules primaires ensemencées dans la structure peuvent être des cellules allogéniques, xénogéniques, hétérologues, ou autologues, vis-à-vis de l'organisme qui doit être traité. Selon une mise en œuvre préférée, il s'agit de cellules autologues, c'est-à-dire qui proviennent du patient, humain ou animal, qui doit être traité. De façon toute préférée, il s'agit donc de chondrocytes humains autologues, qui pourront être réimplantés chez le donneur à l'issue du procédé de l'invention. Il est en de même pour les chondrocytes animaux, par exemple pour les chevaux de course ou les chiens, susceptibles aussi de bénéficier de greffe de chondrocytes. Il peut également s'agir de cellules xénogéniques ou allogéniques, du fait que certaines mesures bien connues de l'homme du métier peuvent être mises en œuvre pour éviter le rejet lors de l'implantation.
Selon un mode de réalisation, il ne s'agit pas de cellules souches embryonnaires humaines.

### La structure tridimensionnelle et le chitosane :

Les cellules sont ensemencées au sein d'une structure tridimensionnelle ou scaffold, ou encore biomatériau, comprenant des fragments ou particules d'hydrogel physique de chitosane pur, ou de dérivé de chitosane. Lesdites particules forment ainsi un échafaudage ou structure compatible et favorable à la formation d'un tissu tridimensionnel, jusqu'à ce que les cellules produisent suffisamment de matrice extracellulaire pour maintenir la structure mécaniquement. Les cellules sont ajoutées après la phase de gélification et de formation des particules d'hydrogel de chitosane. Les cellules sont donc localisées à l'extérieur des fragments ou particules d'hydrogel ; elles restent à la surface des fragments ou particules, elles ne sont ni emprisonnées ni encapsulées dans l'hydrogel, elles n'infiltrent pas non plus les pores de l'hydrogel. Elles peuvent donc circuler librement autour des particules, ainsi que les nutriments et les déchets, durant les différentes étapes du procédé de l'invention. Le mélange réalisé en début de culture entre les particules de chitosane et les cellules permet ainsi de favoriser une bonne distribution des cellules au sein de la structure tridimensionnelle à l'issue de ce procédé. Le chitosane utilisé pour la conception du dispositif ou structure tridimensionnelle est obtenu par exemple par désacétylation de la chitine qui peut provenir de l'exosquelette d'arthropodes (crevettes, insectes, crabe...), de l'endosquelette des céphalopodes (calamars) ou de la paroi cellulaire des champignons. Selon son origine, le chitosane peut être de conformation α (paroi cellulaire de champignons, crevettes, crabes) ou β (calamars) ou ϕ (insectes), ce qui influence fortement ses propriétés biologiques.

Dans le cadre de la présente invention, le chitosane utilisé peut provenir de ces différentes sources, mais il sera préférentiellement utilisé un chitosane d'origine non animale, pour des raisons de biocompatibilité, de faible teneur en endotoxines, de reproductibilité des lots et de conformité aux normes pharmaceutiques. De manière préférentielle, le chitosane utilisé dans le cadre de la présente invention est du chitosane extrait de la paroi cellulaire de champignons, plus particulièrement de champignons de Paris, *Agaricus bisporus.* En effet, dans le cadre des dispositifs implantables, en raison des exigences réglementaires, il est particulièrement avantageux de n'avoir aucune matière qui soit d'origine animale. Le chitosane utilisé dans la présente invention, extrait de champignons de Paris, est conforme aux exigences pharmaceutiques en termes de taux en endotoxines, de résidus microbiologiques et de métaux lourds.

### Succession des étapes :

Le procédé de l'invention se caractérise notamment par la succession de deux étapes, une première étape d'amplification de cellules primaires dans une structure tridimensionnelle et une seconde étape d'induction de la différenciation et de synthèse de matrice extracellulaire (MEC), au sein de cette même structure tridimensionnelle.
L'avantage majeur de ce procédé réside dans le fait qu'il n'est pas nécessaire de changer les cellules de structure, entre l'étape d'amplification et l'étape de re-différenciation avec synthèse de MEC, ni d'ailleurs entre l'étape de re-différenciation et celle d'implantation. Il en découle qu'à aucun moment il n'est nécessaire de procéder à une étape de trypsinisation des cellules. En effet, après l'extraction, les cellules primaires sont ensemencées dans la structure de l'invention, sans étape de prolifération préalable, et donc sans nécessiter de les décoller, notamment par trypsinisation, ou par tout autre moyen susceptible d'endommager la paroi cellulaire. Elles prolifèrent ensuite au sein de la structure, puis sont induites à se redifférencier et à produire de la matrice cartilagineuse, une nouvelle fois sans qu'il soit nécessaire de les décoller, de les trypsiniser, ou de leur faire subir tout autre traitement de nature à endommager la paroi cellulaire.
Le procédé de l'invention permet donc d'obtenir des cellules qui, après l'extraction, n'ont pas été endommagées et n'ont pas subi de conditions de stress, ce qui permet de garantir non seulement une optimisation du nombre de cellules prélevées, en diminuant la mort cellulaire, mais également garantit que les cellules, à l'issue du procédé, ne sont pas dans des programmes de mort cellulaire et n'expriment pas des signaux qui pourraient être néfastes à l'organisme hôte, après réimplantation. A l'issue d'une semaine de culture et d'une façon générale, pendant toute la durée de la culture, le taux de viabilité des cellules est supérieur à 90%, voire supérieur à 93% ou même supérieur à 97%.
De préférence, les deux étapes majeures du procédé de l'invention, c'est-à-dire d'une part l'amplification et d'autre part la re-différenciation et la synthèse de MEC, s'entendent d'étapes distinctes. Les inventeurs ont en effet observé que la capacité à proliférer et la capacité à produire de la MEC étaient préférentiellement des étapes successives pour les cellules telles que les chondrocytes, si bien que les rendements de prolifération et de synthèse de matrice cartilagineuse étaient bien meilleurs quand les deux étapes étaient distinctes. De plus, il existe des milieux qui peuvent favoriser l'une ou l'autre de ces étapes, de manière exclusive, si bien qu'il est préférable de les réaliser l'une après l'autre. De préférence, les deux étapes telles que décrites sont donc non seulement successives mais également distinctes, la seconde étape ne débutant qu'à l'issue de la première étape.

On considère que l'étape d'amplification est distincte de celle de la synthèse de matrice cartilagineuse lorsque le nombre de chondrocytes augmente lors de la première étape et reste relativement stable lors de la seconde étape, avec peu ou pas d'amplification.

On considère que l'étape d'amplification est distincte de celle de la synthèse de matrice cartilagineuse en fonction de la viscosité du milieu : avec l'observation d'une viscosité faible lors de l'étape d'amplification et une viscosité accrue lors de la seconde étape, confirmant la production de matrice extra-cellulaire.
De préférence, lors de la première étape, la synthèse de matrice extracellulaire est faible.
Il est également possible d'observer si une telle matrice est synthétisée en utilisant par exemple des méthodes d'immunohistochimie, telles que celles décrites dans les exemples ci-après.

Le collagène de type II (COLII) est un marqueur caractéristique du cartilage hyalin ; il s'agit d'un homo-trimère de trois chaines α1(II), codées par le gène Col2a1. L'analyse de ce type de collagène est classiquement réalisée pour identifier des chondrocytes différenciés.
Le collagène de type I (COLI), hétérotrimère α1₂ α2₁ produit à partir des gènes Col1a1 et Col1a2 est quant à lui classiquement considéré comme un marqueur de la dé-différenciation des chondrocytes.

Le procédé selon l'invention se caractérise par le maintien de la capacité des cellules qui ont été mises en culture au sein de la structure à se redifférencier en chondrocytes. Par maintien de cette capacité, on entend que la majorité des cellules, à l'issue de la seconde étape, présente un phénotype chondrocytaire, de préférence au moins 60%, de préférence au moins 70%.

Les inventeurs ont en effet observé que la majorité des cellules ensemencées dans la structure telle que décrite, à base de particules d'hydrogel de chitosane ou d'un dérivé, présentent un phénotype chondrocytaire stable durant la deuxième étape du procédé de synthèse de matrice extracellulaire. A l'issue du procédé, les cellules au sein de la composition ne présentent pas ou peu d'expression du collagène de type I et/ou ne le produisent pas au niveau protéique mais expriment un index de différenciation COLII/COLI supérieur à 1. De préférence, les cellules présentes dans cette composition synthétisent des protéines de collagène de type II avec un rapport protéique COLII/COLI supérieur à 1, de préférence supérieur à 1,5 ; et / ou le taux d'ARN messagers de collagène de type II est significativement supérieur au taux d'ARN messagers de type I.

Le procédé selon l'invention tel que décrit plus haut permet de préférence d'obtenir la composition ou gel de cartilage prêt à l'implantation en moins de 40 jours, de préférence en moins de 36 jours, voire même en moins d'une trentaine de jours, par exemple en moins de 28 jours, ou bien moins de 21 jours. Il permet donc, à partir d'une biopsie de chondrocytes ou de cellules souches primaires différenciées en chondrocytes, d'obtenir une composition prête à l'implantation, ayant un nombre suffisant de chondrocytes pour pouvoir réparer la lésion articulaire, et ce en un mois et demi, ou moins d'un mois, ou moins de trois semaines.

A cette fin, l'étape d'amplification est réalisée en une à trois semaines, de préférence en environ deux semaines, de 12 à 16 jours, voire même en moins de deux semaines.
Durant cette étape, la multiplication du nombre de cellules vivantes, par rapport au nombre de cellules initialement ensemencées dans la structure, est d'au moins 4, voire au moins 6 , voire au moins 7 ou supérieur à 7 .
Bien entendu, il peut être décidé de prolonger l'étape de multiplication durant un temps suffisant pour obtenir un nombre déterminé de cellules, à condition de préférence de ne pas atteindre la confluence. La densité des cellules lors de l'ensemencement dans la structure 3D doit bien entendu être adaptée en conséquence.
Selon une mise en œuvre préférée, l'étape de multiplication dure entre 1 et 3 semaines et doit permettre de multiplier les cellules au sein de la structure 3D par un facteur d'au moins 4, voire au moins 6, voire au moins 7.
Par conséquent, une biopsie de 300 à 500 mg de cartilage, comprenant environ 10⁶ à 1,5 x 10⁶ cellules permet d'obtenir, à l'issue de l'étape d'amplification de 4 x 10⁶ cellules à 10,5 x 10⁶ cellules du fait du facteur d'amplification observé par les inventeurs, et ce en 1 à 3 semaines. Un tel nombre de cellules est considéré comme approprié pour l'implantation d'une greffe de chondrocytes.

La seconde étape, liée à la re-différenciation s'accompagnant de synthèse de matrice extracellulaire, peut avoir une durée variable.
En vue de la réimplantation du gel de cartilage, il est cependant préférable qu'une telle étape dure entre 2 et 4 semaines, de préférence environ trois semaines, ou bien moins. La durée de cette seconde étape peut éventuellement être ajustée en fonction du mode de réimplantation choisi.
A l'issue de la mise en œuvre du procédé de l'invention, il est donc obtenu une composition ou gel de cartilage, comprenant des cellules chondrocytaires, réparties dans une matrice cartilagineuse nouvellement synthétisée, et qui sont aptes à poursuivre la synthèse de tissu cartilagineux, au sein d'une structure 3D composée de particules d'hydrogel physique de chitosane ou d'un dérivé de chitosane, telle que ladite composition ou gel de cartilage est directement implantable chez un être humain ou un animal, notamment pour combler une lésion articulaire cartilagineuse, par exemple suite à des lésions traumatiques et limités du cartilage articulaire, ou des lésions de type arthrose débutante superficielle, ou des lésions plus profondes de type ostéochondrale.

On obtient donc à l'issue du procédé un kit ou gel de cartilage prêt à l'injection ou l'implantation in vivo chez un être humain ou un animal. Si la structure 3D comprenant les particules d'hydrogel de chitosane ou le dérivé de chitosane, peut être en partie biodégradée lors du procédé, elle l'est de préférence très peu, de préférence moins de 50% de la structure 3 D initiale à base de particules d'hydrogel de chitosane avant l'ensemencement.
En fonction de la durée des étapes du procédé de l'invention, notamment de l'étape de synthèse de MEC, il est également envisageable que la composition de l'invention ou gel de cartilage puisse être injectable chez un être humain ou un animal. A cet effet, la durée de la seconde étape de synthèse de MEC sera réduite de quelques jours, afin d'assurer que la composition reste injectable.
A l'inverse, notamment lorsqu'il sera souhaitable d'obtenir un implant ayant une forme bien déterminée, l'étape de synthèse de MEC sera ajustée afin d'obtenir la consistance souhaitée pour ensuite pouvoir ajuster la forme de la composition à celle souhaitée. Il est également envisageable de produire directement la structure dans un contenant ayant la forme souhaitée et de réaliser les différentes étapes du procédé dans ce contenant de telle façon qu'à l'issue de l'étape de synthèse de matrice cartilagineuse, le gel de cartilage ait la forme induite par le contenant.
La composition telle que décrite plus haut comprend du cartilage hyalin, synthétisé par les cellules durant la seconde étape du procédé, d'où son appellation de gel de cartilage.
Un atout majeur de la présente invention réside en effet dans le caractère directement implantable de la composition obtenue à l'issue du procédé. Il n'est notamment pas nécessaire de changer les cellules de structure 3D, donc pas nécessaire de leur faire subir un quelconque traitement. Il n'est pas nécessaire non plus de s'assurer de la disparition de la structure, ni d'attendre une telle dégradation. Au contraire, selon le procédé de l'invention, la structure à base de chitosane est toujours présente à l'issue de l'étape de synthèse de MEC et fait partie de la composition, ou néo-tissu, destiné à être implanté. En effet, une telle structure sert d'échafaudage assurant le maintien des cellules dans un environnement adapté tel qu'il permette, après implantation, la poursuite de la synthèse de MEC, afin de parfaitement s'insérer au niveau de la lésion, par exemple articulaire. La structure de chitosane n'a donc pas la seule vocation à favoriser la synthèse de MEC in vitro, mais également, après réimplantation, à soutenir les cellules implantées ; elle participe donc à la structure du néo-tissu réimplanté.
La structure assure par ailleurs une distribution spatiale optimale des cellules ensemencées, elle permet donc une distribution harmonieuse de la MEC synthétisée, durant le procédé de l'invention, et aussi après réimplantation.

Le nombre de cellules implantées dans la structure de l'invention est variable en fonction de la taille de la lésion qu'il est souhaité de combler, et également en fonction du nombre de cellules qu'il est envisageable de prélever aux fins d'ensemencement. De préférence cependant, au moins environ 10⁵ cellules primaires sont ensemencées, notamment au moins environ 6×10⁵ cellules primaires, ou au moins 10⁶ cellules primaires, de préférence des chondrocytes primaires humains, canins ou équins; à l'issue du procédé de l'invention, la composition finale comprend de préférence au moins 3x10⁵ chondrocytes, ou au moins 6×10⁶ chondrocytes, voire plus encore. En vue de l'obtention d'une composition directement implantable dans une lésion cartilagineuse, il sera de préférence ensemencé 10⁶ à 1.5 x 10⁶ cellules permettant d'obtenir, à l'issue de l'étape d'amplification de 4 à 10,5 x 10⁶ cellules.
Selon une mise en œuvre de l'invention, les chondrocytes au sein de la composition sont à une concentration d'environ au moins 10⁶ cellules/g, de préférence environ au moins 6×10⁶ cellules/g de structure 3D au moment de la mise en culture.

### Milieux de culture :

Selon un mode de réalisation particulièrement préféré, il est utilisé deux milieux de culture différents. L'un pour la première étape d'amplification et l'autre pour la seconde étape d'induction de la re-différenciation et de synthèse de MEC ; le passage de l'une à l'autre des étapes étant de ce fait réalisé par le changement de milieu, les deux milieux étant distincts.
Il est notamment envisagé d'utiliser lors de la première étape un milieu favorisant la prolifération des cellules, sans induire la synthèse de MEC. La prolifération des cellules s'accompagne généralement d'un phénomène de dé-différenciation ; on utilisera toutefois un milieu qui préserve leur capacité à se redifférencier en chondrocytes à l'issue de l'étape de prolifération. Les inventeurs ont en effet mis en évidence le fait que, au sein d'une structure tridimensionnelle, il était préférable de procéder à une étape d'intense amplification, sans pour autant induire la synthèse de MEC.

En effet, les structures tridimensionnelles et le chitosane étaient connus avant l'invention pour leur capacité à favoriser le phénotype chondrocytaire en limitant la dédifférenciation, lors de l'étape de synthèse de matrice extracellulaire. De manière parfaitement inattendue, les inventeurs ont montré qu'il était possible d'amplifier les chondrocytes sans induire de synthèse massive de matrice extracellulaire, au sein d'une structure tridimensionnelle à base d'hydrogel de chitosane ou d'un dérivé.

Un milieu particulièrement adapté pour l'étape de prolifération est un milieu induisant l'amplification, notamment un milieu comprenant du facteur de croissance fibroblastique (FGF-2, «fibroblast growth factor»), et accessoirement aussi de l'insuline, correspondant à un milieu dit « FI » illustré dans la partie expérimentale (Claus et al, 2012). Le FGF-2 est de préférence présent entre 2 et 10 ng/mL et l'insuline entre 2 et 10 µg/mL. Cependant, d'autres milieux de culture, bien connus de l'homme du métier, peuvent également être utilisés, notamment tous les milieux de culture généralement utilisés pour ce genre de cellules, mais en monocouches. Du fait de la structure tridimensionnelle et du chitosane, les cellules ensemencées dans la structure de l'invention conservent principalement leur morphologie ronde et leur capacité à ultérieurement se redifférencier en chondrocytes.
Pour le milieu de culture de la seconde étape, on utilisera de préférence des milieux classiquement utilisés afin de favoriser la différenciation ou re-différenciation de chondrocytes dédifférenciés et permettant la synthèse de MEC spécifique. De tels milieux sont bien connus de l'homme du métier. Un milieu tout particulièrement préféré comprend de la BMP-2 (Bone morphogenetic protein 2) ; de préférence, le milieu est celui utilisé dans la partie expérimentale, composé notamment de BMP-2 et d'insuline, et de préférence également de la triiodothyronine T3 (Liu et al, 2007, Claus et al, 2012), correspondant à un milieu dit « BIT ». La BMP-2 est de préférence à une concentration comprise entre 100 et 500 ng/mL, l'insuline entre 2 et 10 µg/mL et la triiodothyronine T3 entre 50 et 250 mM.
La BMP-2 sera choisie de préférence de la même espèce que les cellules utilisées, soit une BMP-2 humaine pour des cellules humaines. Il en est de même pour les cellules animales.
Aussi bien pour le milieu de culture de la première étape que celui de la seconde, on privilégiera des milieux de culture qui ne s'opposent pas à la réimplantation ultérieure de la composition, à l'issue de l'étape de synthèse de MEC. On évitera notamment les composés risquant de générer des réactions de rejet, non compatibles avec la réglementation relative aux dispositifs implantables.
Accessoirement, il peut être envisagé des étapes d'élimination du milieu de culture avant l'injection ou la réimplantation à l'issue des deux étapes mentionnées plus haut.
La première et la seconde étape peuvent, indépendamment l'une de l'autre, se dérouler en conditions normoxie ou hypoxie.

### Réalisation de l'hydrogel de chitosane :

L'hydrogel de chitosane pur, ou de dérivé de chitosane, est réalisé à partir de chitosane, de préférence extrait de champignons et dont la masse molaire moyenne en poids (Mw), sera préférentiellement supérieure à 150 kDa (i.e. 150 000 g/mol) en vue de favoriser le procédé de gélification physique par la présence de longues chaines macromoléculaires. Elle sera de préférence comprise entre 150 et 220 kDa.
Si le chitosane est de masse molaire largement différente, notamment s'il est extrait d'une autre source, le procédé de réalisation de l'hydrogel physique de chitosane tel que décrit dans la partie expérimentale pourra être aisément adapté par l'homme du métier, en utilisant des techniques bien connues.
De plus, il est également possible d'utiliser du chitosane, pour l'hydrogel, qui ait un degré variable d'acétylation ; de préférence néanmoins, le degré d'acétylation du chitosane est compris entre 5% et 60%, de préférence supérieur à 25%, par exemple entre 25 et 60%, ou entre 28 et 40%. Ce degré d'acétylation induit en effet un environnement favorable aux cellules, conduisant à une bonne adhésion entre l'hydrogel formé et les cellules, et de bons résultats en chondrogenèse.
En vue de la formation de l'hydrogel, on utilise de préférence une solution de chitosane dont la concentration est suffisamment élevée pour permettre l'enchevêtrement des chaînes macromoléculaires, et ainsi favoriser la gélification physique. L'hydrogel est en effet obtenu dans le cadre de l'invention par un processus entièrement physique, sans aucun réticulant chimique. La concentration du chitosane en solution peut être comprise entre 0,5-4% (w/w), de préférence supérieure à 1,5 %, voire 2%. Préférentiellement, le chitosane, ou le dérivé de chitosane a une concentration en masse dans l'hydrogel comprise entre 3,4 et 4,2% avant neutralisation.

Plusieurs procédés de gélification du chitosane peuvent être utilisés dans le cadre de la présente invention. On peut notamment citer les procédés suivants, particulièrement bien adaptés comme la gélification physique par voie gazeuse (ammoniaque) ou la gélification physique en milieu hydroalcoolique ou aqueux.
Préférentiellement, l'hydrogel utilisé dans le cadre de la présente invention est obtenu par un procédé d'évaporation en milieu alcoolique, tel qu'illustré à l'exemple 1 dans la partie expérimentale ; un tel procédé est également appelé gélification hydroacloolique.
L'hydrogel obtenu a de préférence entre 3 et 5 mm d'épaisseur.
La taille des pores de l'hydrogel obtenu doit être à la fois inférieure à la taille des cellules et à la fois suffisante pour permettre la libre diffusion des nutriments et l'élimination des déchets. Les modes de réalisation décrits ci-dessus et mis en œuvre dans la partie expérimentale permettent d'obtenir une telle taille de pores. Il est à noter que, dans le cadre de la présente invention, l'hydrogel de chitosane ou de dérivé de chitosane est tel que la taille de ses pores ne permet pas la pénétration des cellules à l'intérieur de l'hydrogel. Les cellules ensemencées au sein de la structure tridimensionnelle selon l'invention prolifèrent donc sans pénétrer dans l'hydrogel. Du fait que les cellules ne se multiplient pas dans les pores de l'hydrogel mais qu'elles peuvent librement circuler autour des particules d'hydrogel, permet d'obtenir une répartition, ou distribution, homogène des cellules dans la structure, sans les importants gradients cellulaires rapportés dans Correia et al, 2011.
L'homme du métier est en mesure de déterminer la taille des pores d'un hydrogel et d'ajuster les paramètres de sa réalisation afin de s'assurer que la taille des pores est suffisamment petite pour empêcher la pénétration des cellules, notamment de chondrocytes, tout en permettant néanmoins la libre diffusion des nutriments.
Afin d'obtenir les particules d'hydrogel constituant la base de la structure tridimensionnelle de l'invention, l'hydrogel ainsi obtenu est manipulé par tout moyen adéquat, bien connu de l'homme du métier ; par ce biais, l'hydrogel est fragmenté en particules.
Les particules d'hydrogel ainsi obtenues sont de forme irrégulière, mais présentent de préférence une distribution de taille relativement homogène, c'est-à-dire que 50% des particules ont une taille comprise entre -20% et +20% de la taille moyenne. Selon une mise en œuvre préférée, les particules ont une taille moyenne comprise entre 10 µm et 1500µm ; de préférence entre 200 µm et 1200 µm (1, 2 mm), et de préférence encore entre 400 µm et 700 µm. Par taille des particules, on entend la longueur de l'arête, si les particules sont assimilés à des rectangles, la longueur du diamètre le plus grand si les particules sont assimilés à des ellipses. Les particules sont de préférence assimilables à des ellipses.
Les inventeurs ont mis en évidence des résultats meilleurs pour des particules d'une taille moyenne supérieure à une centaine de microns, notamment au-delà de 60 µm, voire au-delà de 400 µm et inférieure à 1,2 mm.
Une certaine variabilité des tailles de particules d'hydrogel de chitosane semble être favorable à la culture des chondrocytes.
Selon une mise en œuvre préférée, l'hydrogel est d'abord obtenu par gélification par voie hydroalcoolique, puis fragmenté par tout moyen approprié.

### Conception de la structure tridimensionnelle :

La structure tridimensionnelle selon l'invention comprend donc des particules d'hydrogel physique de chitosane ou d'un de ses dérivés, comme détaillé plus haut, constituant une structure tridimensionnelle au sein de laquelle les cellules sont ensemencées, ou migrent naturellement. Selon un mode de réalisation préféré, les cellules sont mélangées avec les particules d'hydrogel. L'hydrogel physique de chitosane pur ou de dérivé de chitosane est de préférence uniquement constitué de chitosane ou d'un dérivé, et d'eau avec une teneur de préférence au moins 70%, de préférence au moins 80%. Notamment, il n'entre dans la composition de cet hydrogel, ni agent chimique de réticulation, ni autre polymère, notamment polysaccharide ou dérivé, mis à part le β-glucan naturellement associé au chitosane. L'important pourcentage en eau assure qu'il s'agit bien d'un hydrogel de chitosane et non d'une structure de type éponge ou autre, obtenue par lyophilisation d'une solution de chitosane. Il est à noter que l'important pourcentage en eau dans l'hydrogel permet de mimer au mieux l'environnement naturel des chondrocytes, le tissu cartilagineux comprenant 80% d'eau environ.

Selon une mise en œuvre particulièrement préférée, une molécule anionique est ajoutée aux particules d'hydrogel de chitosane ou de dérivé de chitosane permettant de renforcer les propriétés mécaniques et biologiques de la structure tridimensionnelle. Cette molécule n'entre pas dans la composition de l'hydrogel, il s'agit d'un constituant ajouté après gélification de l'hydrogel de chitosane ou de dérivé de chitosane, et de préférence après réalisation des particules d'hydrogel. La molécule anionique interagit donc uniquement à la surface des particules d'hydrogel selon l'invention, favorisant ainsi la formation de particules de chitosane chevelues par les chaines linéaires de cette molécule anionique.
La molécule anionique associée à la surface des particules de chitosane, se trouve de préférence sous forme de polymère, il s'agit par exemple d'acide hyaluronique, ou de chondroitine sulfate. Les chaines de chitosane présentant des charges positives, dues aux groupements amines sous forme protonée NH³⁺, et les chaines de cette molécule anionique interagissent par des liaisons électrostatiques, formant ainsi un complexe stable en milieu physiologique (pH compris entre 5 et 8, et plus généralement compris entre 6 et 7). La molécule anionique permet ainsi de réticuler de manière électrostatique les fragments ou particules d'hydrogel en interagissant avec les chaines cationiques du chitosane en périphérie des particules, renforçant ainsi les propriétés mécaniques de la structure tridimensionnelle ou 'scaffold' au sein duquel les cellules sont ensemencées.
Il est à noter que la molécule anionique peut être ajoutée à la structure tridimensionnelle avant l'ensemencement avec les cellules primaires, et donc être présente durant l'étape de prolifération et de synthèse de MEC, et donc également lors de l'implantation ultérieure de la composition. Dans le cadre de l'invention, il est également envisagé de procéder à l'ensemencement des cellules primaires dans une structure tridimensionnelle dépourvue de la molécule anionique et de rajouter cette dernière soit au cours de la première étape de multiplication, soit à l'issue de la première étape au moment de débuter la seconde étape de re-différenciation, ou bien à l'issue de la seconde étape de re-différenciation et synthèse de MEC, avant l'implantation.
La molécule anionique sera préférentiellement de l'acide hyaluronique, un des constituants du liquide synovial, connu pour ses propriétés de chondroprotection et favorable à la chondrogénèse. Il est ainsi ajouté la quantité d'acide hyaluronique nécessaire pour modifier les caractéristiques visco-élastiques de la structure tridimensionnelle. Les interactions électrostatiques interviennent entre les groupements amines sous forme protonée NH3+ du chitosane et les groupements carboxyliques de l'acide hyaluronique.
Il peut s'agir également d'un dérivé d'acide hyaluronique ou un complexe d'acide hyaluronique. La proportion relative de la molécule anionique vis-à-vis de l'hydrogel de chitosane est préférentiellement comprise entre 1 et 10%, de préférence entre 1 et 3%.
L'acide hyaluronique peut être d'origine animale, par exemple par extraction de crête de coq ou d'origine non animale, obtenu par fermentation bactérienne. Dans le cadre de la présente invention, l'acide hyaluronique sera tout préférentiellement choisi d'origine bactérienne. En effet, l'acide hyaluronique obtenu par fermentation bactérienne est connu pour ses meilleures propriétés de biocompatibilité, évitant ainsi les allergies et les rejets, de reproductibilité des lots et de conformité aux normes pharmaceutiques. L'acide hyaluronique utilisé dans le cadre de la présente invention est conforme aux normes pharmaceutiques.
Sa masse moléculaire en poids est de préférence de 50 kDa à 4 MDa, elle sera choisie préférentiellement supérieure à 500 kDa, de préférence entre 500 kDa et 2 MDa, par exemple entre 1 MDa et 2MDa.

De manière tout particulièrement préférée dans le cadre de la présente invention, les constituants de la structure tridimensionnelle, qui sont des particules d'hydrogel de chitosane ou de dérivés de chitosane, avec ou sans addition de chaines de composé anionique, doivent être résorbables in vivo. Afin d'obtenir une telle propriété, il est important qu'aucun des constituants de la structure tridimensionnelle ne s'oppose à son caractère résorbable.
De préférence, l'association des particules d'hydrogel de chitosane ou de dérivés de chitosane, avec ou sans les chaines de composé anionique, se résorbera au bout de plusieurs semaines une fois implantée, par exemple après au moins deux semaines, de préférence au moins 4 semaines. Il est généralement préféré que le temps de résorption n'excède pas 6 mois, de préférence même n'excède pas 4 mois. Selon le type d'application envisagée, le temps de résorption peut être ajusté par l'homme de métier.

Il est important de noter que les compositions selon la présente invention peuvent être adaptées en termes de forme, de diamètre, de concentration, de teneur en fonction des différentes applications envisagées. Notamment, la structure tridimensionnelle formée de particules d'hydrogel de chitosane ou de dérivé de chitosane, avec ou sans les chaines de composé anionique, peut être élaborée afin que sa forme corresponde à celle de la lésion observée dans laquelle elle sera réimplantée à l'issue du procédé selon l'invention.
Néanmoins, aucune étape de déshydratation n'est nécessaire ou souhaitée avant la réimplantation.

Comme précisé plus haut, le procédé permet d'obtenir une composition ou gel de cartilage prêt à l'implantation ou l'injection in vivo, notamment chez un être humain, ou un animal tel que le chien ou le cheval.
Cependant, avant son implantation, il peut être envisagé de changer le milieu, ou bien d'ajouter des composés additionnels, notamment solubles au sein de la structure tridimensionnelle. Par exemple, il est envisageable d'ajouter des composés pharmaceutiques tels que des agents anti-inflammatoires, des anesthésiques, analgésiques, corticostéroïdes, des vitamines, des minéraux, des composés visant à réduire la réponse immunitaire, et / ou à favoriser la greffe, tout ou partie de ces composés ou une combinaison de ces composés, sans que cette liste ne soit limitative.

Selon un second aspect, la présente invention concerne une structure tridimensionnelle formée de particules d'hydrogel physique de chitosane ou d'un dérivé de chitosane, et d'une molécule anionique associée à ces particules. Une telle matrice peut avantageusement être utilisée pour ensemencer des chondrocytes en les faisant proliférer puis synthétiser de la matrice extracellulaire, tout particulièrement de la matrice caractéristique du cartilage hyalin, notamment pour une utilisation selon la présente invention, avant d'être implantée ou injectée, sous forme de gel de cartilage, notamment au sein d'une lésion articulaire. Comme démontré dans le cadre de la présente invention, une telle structure permet en effet d'assurer non seulement la prolifération de cellules telles que des chondrocytes, et fournit également un environnement particulièrement favorable à la synthèse de matrice de cartilage hyalin. L'invention concerne également une composition implantable ou injectable, comprenant cette structure tridimensionnelle et des chondrocytes différenciés aptes à synthétiser du tissu cartilagineux. Il est à noter que la composition, ou gel de cartilage, comprend aussi de la matrice cartilagineuse, synthétisée par les chondrocytes qu'elle contient.

Les différents éléments mentionnés sont tels que décrits pour le premier aspect de l'invention, notamment la structure tridimensionnelle, le chitosane ou son dérivé, l'hydrogel, les particules, la molécule anionique, en regard du procédé de l'invention. Les mises en œuvre préférées dans le cadre de ce premier aspect le sont également dans le cadre de ce second aspect. En particulier, le chitosane est de préférence un chitosane obtenu à partir de champignons, et plus particulièrement extrait de la paroi cellulaire de champignons de Paris, *Agaricus bisporus.* Les particules ont les tailles spécifiées plus haut, soit entre 10 µm et 1200 µm, de préférence entre 400 et 700 µm en moyenne. La structure est donc préférentiellement une structure 3D formée de particules d'hydrogel physique de chitosane, de taille moyenne comprise entre 400 µm et 700 µm, où ledit chitosane est extrait à partir de champignons de Paris.
Concernant la molécule anionique, comme précisé pour le procédé de l'invention, il s'agit de préférence d'une molécule polymérique et tout préférentiellement d'acide hyaluronique ou d'un dérivé d'acide hyaluronique ou un complexe d'acide hyaluronique, et plus spécifiquement d'acide hyaluronique provenant de fermentation bactérienne.
Les chondrocytes différenciés, présents au sein de la composition de l'invention, sont par exemple des chondrocytes articulaires. De manière toute préférée, il s'agit de chondrocytes humains ou animaux, notamment canins ou équins. Les chondrocytes sont des chondrocytes différenciés, présentant un phénotype chondrocytaire, notamment ayant un index de différenciation COLII/COLI supérieur à 1. De préférence, les chondrocytes présents dans cette composition synthétisent principalement des protéines de collagène de type II avec un rapport protéique COLII/COLI supérieur à 1, de préférence supérieur à 1,5 ; et / ou le taux d'ARN messagers de collagène de type II est significativement supérieur au taux d'ARN messagers de type I, avec un rapport du niveau transcriptionnel COLII/COLI des cellules supérieur à 1, par exemple supérieur à 100, ou supérieur à 1000.

Selon une mise en œuvre de l'invention, la proportion relative de chondrocytes au sein de la composition correspond à une concentration comprise entre 10⁶ et 10⁷ cellules/g de structure hydrogel 3D, au moment de la mise en culture.
La composition de l'invention peut comprendre également d'autres composés ou molécules, et notamment de la matrice extracellulaire (MEC).
De manière préférée, une composition telle que décrite, ou gel de cartilage, est susceptible d'être obtenue par la mise en œuvre du procédé de l'invention, notamment par la mise en culture des cellules au sein de la structure tridimensionnelle, puis leur prolifération, suivie de leur re-différenciation accompagnée de synthèse de MEC.
Il peut être envisagé également d'ajouter des composés additionnels, notamment solubles, par exemple choisis parmi des agents anti-inflammatoires, des anesthésiques, analgésiques, corticostéroides, des vitamines, des minéraux, des composés visant à réduire la réponse immunitaire, et / ou à favoriser la greffe, tout ou partie de ces composés ou une combinaison de ces composés sans que cette liste ne soit limitative.

Comme décrit dans le cadre du procédé de l'invention, la structure tridimensionnelle telle que décrite est avantageusement résorbable, notamment biorésorbable in vivo. Les propriétés de l'hydrogel de chitosane ou du dérivé de chitosane seront choisies en fonction de l'échelle de temps souhaitée jusqu'à la résorption intégrale de la structure tridimensionnelle, une fois la composition implantée. De préférence, le temps de résorption sera adapté afin qu'une telle résorption s'effectue concomitamment avec la synthèse de la matrice cartilagineuse par les chondrocytes présents dans la composition ; de manière préférée, le temps de résorption sera ajusté de telle façon que la matrice cartilagineuse formée par les chondrocytes se substitue intégralement à la structure tridimensionnelle d'hydrogel de chitosane ou de dérivé de chitosane.

Selon un autre aspect de l'invention, la composition telle qu'obtenue à l'issue du procédé de l'invention, ou telle que décrite selon le second aspect de l'invention, est pour une utilisation thérapeutique et/ou chirurgicale, notamment pour une utilisation comme implant ou greffe dans la réparation ou la reconstruction de tissu cartilagineux, ou dans le traitement de l'arthrose, et plus généralement dans le traitement de toute pathologie caractérisée par une dégradation ou une disparition du tissu cartilagineux, notamment lésion cartilagineuse, par exemple suite à des lésions traumatiques et limités du cartilage articulaire, ou des lésions plus profondes de type ostéochondrale. Une telle composition pour une utilisation in vivo est notamment envisagée en chirurgie, en rhumatologie, ou comme vecteur de principe actif. La composition, ou gel de cartilage, est implantable par arthroscopie.
Une utilisation spécialement envisagée est la greffe de chondrocytes. De préférence, les chondrocytes présents au sein de la composition à greffer sont des cellules autologues ou allogéniques, de préférence il s'agit de chondrocytes humains, canins ou équins.

Selon encore un autre aspect, la présente invention concerne également une structure tridimensionnelle formée de particules ou fragments d'hydrogel physique de chitosane pur ou d'un dérivé de chitosane, et son utilisation pour l'ensemencement de cellules in vitro, notamment aux fins de prolifération et de synthèse de matrice extracellulaire, notamment pour la mise en œuvre du procédé selon le premier aspect de la présente invention. La structure tridimensionnelle est telle que décrite précédemment; il en va de même pour l'hydrogel de chitosane ou de dérivé de chitosane. Ce dernier est de préférence extrait de champignons de Paris comme explicité plus haut, ayant une masse molaire moyenne en poids comprise de préférence entre 150 et 220 kDa. Les particules d'hydrogel ont de préférence une taille moyenne comprise entre 200 µm et 1, 2 mm, et de manière toute préférée entre 400 et 700 µm. Une molécule anionique est de préférence ajoutée aux particules d'hydrogel de la structure tridimensionnelle ; il s'agit préférentiellement d'un polymère anionique, tout particulièrement d'acide hyaluronique ou un dérivé d'acide hyaluronique ou un complexe d'acide hyaluronique, notamment obtenu par fermentation bactérienne.
Toutes les mises en œuvre préférées détaillées plus haut concernant les éléments de la structure tridimensionnelle sont également applicables à cet aspect de l'invention, et tout particulièrement les caractéristiques suivantes : les particules d'hydrogel ont de préférence une taille comprise entre 200 µm et 1,2 mm, de préférence entre 400 et 700 µm, et/ou le chitosane a une masse molaire moyenne en poids comprise de préférence supérieure à 50 kDa, de préférence entre 150 et 220 kDa, et/ ou le chitosane a un degré d'acétylation compris entre 5 et 60%, de préférence entre 28 et 40%, et/ou l'acide hyaluronique a une masse molaire moyenne en poids comprise entre 50 kDa et 4MDa, de préférence entre 1 et 2 MDa.

La proportion d'acide hyaluronique vis-à-vis d'hydrogel de chitosane est de préférence comprise entre 1 et 10%, de préférence entre 1 et 3%. Comme décrit plus haut, cette structure tridimensionnelle est avantageusement utilisée pour l'ensemencement ou mise en culture de cellules primaires, notamment de chondrocytes primaires, ou de cellules souches primaires différenciées en chondrocytes, notamment cellules souches mésenchymateuses. Cependant, d'autres types de cellules peuvent être cultivées dans cette structure tridimensionnelle, notamment des cellules osseuses, des fibroblastes, des kératinocytes, ou des combinaisons de certaines de ces cellules, sans que cette liste ne soit limitative. Les présents inventeurs ont en effet mis en évidence que cette structure tridimensionnelle fournissait une architecture tridimensionnelle particulièrement favorable aux cellules, que ce soit dans des phases de prolifération ou de multiplication, que dans des phases de synthèse de matrice extra-cellulaire. Par ailleurs, comme décrit plus haut, cette structure tridimensionnelle est biodégradable et biorésorbable, et peut donc être implantée in vivo, chez l'homme ou l'animal, une fois ensemencée par des cellules.

Dans le cadre de la présente invention, il est également envisagé d'implanter in vivo une telle structure tridimensionnelle de l'invention, c'est-à-dire une structure tridimensionnelle comprenant des fragments ou particules d'hydrogel physique de chitosane ou de dérivé de chitosane, réticulées de manière électrostatique par une molécule anionique, de préférence un polymère, notamment de l'acide hyaluronique ou dérivé d'acide hyaluronique ou un complexe d'acide hyaluronique, ladite structure étant dépourvue de cellules, de telle façon que la structure 3D soit précisément colonisée, in vivo, par des cellules.
La structure tridimensionnelle telle que décrite dans le cadre des différents aspects de cette invention, est préférentiellement stérilisée avant tout ensemencement.

### FIGURES :

Fig.1 : montre un cliché d'hydrogel physique de chitosane pur, obtenu par Cryo-Microscopie électronique à balayage.
Fig.2 : montre un cliché d'hydrogel physique de chitosane pur déshydraté, obtenu par Microscopie électronique à balayage.
Fig.3 : montre un cliché de particules d'hydrogel physique de chitosane pur, après traitement à l'éosine, obtenu par Microscopie optique.
Fig.4 : montre le taux de survie des cellules ensemencées dans une structure 3D de type M1, en fonction de la densité initiale de chondrocytes, mesuré avec le kit Live and Dead sur des fractions de cultures à 7 jours (en cours d'amplification, en milieu FI). Le dénombrement des cellules mortes (en noir) et des cellules vivantes (grisées) est réalisé avec le logiciel ImageJ à partir de clichés pris en microscopie à fluorescence au x20. Le pourcentage de cellules mortes est calculé pour chaque condition en faisant le rapport cellules mortes / cellules totales.
Fig.5 : montre l'évolution en fonction du temps de la population de chondrocytes au sein de structures 3D à base de particules d'hydrogel physique de chitosane extrait de champignons, supplémentées ou pas par l'acide hyaluronique, avec des tailles de particules différentes, ou au sein de structures 3D à base de particules d'hydrogel physique de chitosane extrait de calmar, ou encore de chondrocytes cultivés en monocouches, dans les conditions de culture « FI ». Le nombre de cellules est porté en ordonné, le temps de culture en jours est porté en abscisse
Fig.6 : montre l'évolution de la population de chondrocytes en fonction du temps pour différentes densités initiales de chondrocytes cultivés en monocouches, dans les conditions de culture « FI », confirmant que la population cellulaire obtenue est identique à partir de 7 jours quelque soit la densité initiale.
Fig.7 : montre des clichés de microscopie optique en fonction du temps, de chondrocytes cultivés au sein de structures tridimensionnelles de particules d'hydrogel pur (M1).
   La Fig.7A représente les cellules à l'issue de l'étape d'amplification, en milieu FI, 14 jours après l'ensemencement.
   La Fig.7B représente les cellules au cours de l'étape de synthèse de MEC, en milieu BIT, 24 jours après l'ensemencement, soit 10 jours après l'induction de la re-différenciation et de la synthèse de MEC. Le facteur d'agrandissement est x 20.
Fig.8 : montre la quantité des ARN messagers du collagène de type I et du collagène de type II relativement au gène GAPDH, mesurée par RT-PCR quantitative, pour des chondrocytes cultivés au sein d'une structure 3D (M1) avec plusieurs densités cellulaires initiales en comparaison avec la technique monocouche, au bout de 35 jours de culture.
Fig.9 : montre le rapport des ARN messagers des gènes de COLII/COLI, obtenu par RTC-PCR quantitative, des chondrocytes ensemencés au sein d'une structure 3D (M1) avec plusieurs densités cellulaires initiales en comparaison avec la technique monocouche, au bout de 35 jours de culture.
Fig.10 : montre l'analyse par Western Blot, des taux protéiques du collagène de type I et du collagène de type II, pour des chondrocytes cultivés en structure tridimensionnelle M1 comparativement à des chondrocytes cultivés en monocouches, au bout de 35 jours. Le niveau d'expression de l'actine sert de contrôle.
Fig.11 : montre l'analyse par immunohistochimie de chondrocytes cultivés en structure 3D M1 et M2, comparativement à ceux cultivés en monocouches (x20) (MC) au bout de 35 jours, pour une même densité cellulaire initiale (6.10⁵cellules) par colorations HES et SO ainsi que immunomarquages du collagène de type I et du collagène de type II.

### EXEMPLES :

### Exemple 1 : Synthèse d'hydrogel physique à base de chitosane.

Le chitosane utilisé est d'origine non animale, il est extrait de la paroi cellulaire de champignons de Paris, Agaricus bisporus. Sa masse molaire moyenne en poids (Mw) est de 170 g/mol; et son degré d'acétylation (DA) de 32%. Il est utilisé sous forme de poudre.
Le chitosane pur est mis en solution dans une solution acide d'acide acétique (1% dans eau) en quantité stoechiométrique avec les groupements amine du chitosane. La solution est agitée à température ambiante jusqu'à dissolution complète du chitosane, soit pendant au moins 3h, de préférence au moins 6h.
Puis on ajoute le 1,2 propanediol en quantité identique à celle d'acide acétique et on laisse agiter pendant au moins 30 min, de préférence 1h à température ambiante. Le mélange peut ensuite être dégazé à température ambiante, ou sous vide si nécessaire, si la solution présente beaucoup de bulles d'air.
La solution est ensuite coulée dans des récipients, de type plaque multi-puits ou boites de pétri de 3cm de diamètre, puis est laissée au repos, de préférence pendant une nuit. La solution est alors placée dans une étuve sous vide, préférentiellement à 50°C, pendant le temps nécessaire à la formation du gel, de préférence au moins 20 heures.
L'étape de gélification peut également être réalisée à température ambiante, mais nécessite alors des temps plus longs (5-8 jours en fonction des caractéristiques intrinsèques du chitosane).
L'épaisseur de la solution avant gélification peut être comprise entre 2 et 7 mm, préférentiellement entre 3-6 mm, pour favoriser l'évaporation et les interactions de type hydrophobes pour une bonne prise en gel.

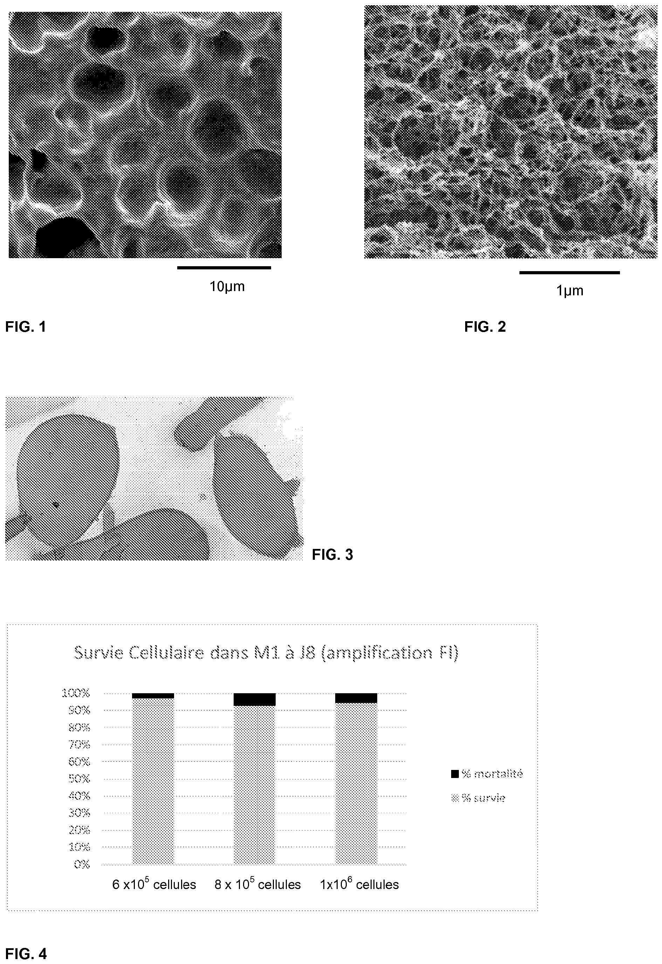

L'hydrogel physique obtenu est alors neutralisé en milieu basique, avec une solution de soude NaOH 0,1N, de préférence pendant 1h. On procède ensuite à plusieurs lavages à l'eau, de préférence stérile. Les lavages durent de préférence environ 1 heure chacun pour retirer les excès d'alcool et ramener l'hydrogel à un pH neutre. Il a été réalisé en général au moins 6 lavages.
Le gel ainsi obtenu a une teneur en eau d'environ 80% en masse. La concentration finale en masse en chitosane dans l'hydrogel est comprise entre 1% et 4,5% avant neutralisation, préférentiellement entre 3,4 et 4,2% avant neutralisation.
Il est important de contrôler les conditions de température et d'humidité lors de la synthèse des hydrogels à base de chitosane, tout particulièrement quand celui-ci est extrait de champignons, préférentiellement dans des conditions de température ambiante inférieures à 25°C.
L'hydrogel obtenu à l'issue de ces différentes étapes a une épaisseur de 3 à 6 mm, de préférence entre 4 et 5 mm d'épaisseur, il est de couleur blanche translucide, et ses surfaces sont lisses et régulières. Cependant son aspect peut varier en fonction des propriétés intrinsèques du chitosane de base, notamment du degré d'acétylation, de la masse molaire et la concentration. Il se présente sous la forme d'un bloc visco-élastique, dont les propriétés mécaniques dépendant des caractéristiques intrinsèques du chitosane de départ, notamment une nouvelle fois le degré d'acétylation, la masse molaire et la concentration.
L'hydrogel obtenu est aisé à manipuler, il se détache sans difficulté et sans se déchirer de la surface plane sur laquelle il a été réalisé.
L'observation en Microscopie Electronique à Balayage conventionnel de l'hydrogel déshydraté montre une structure fibrillaire 3D, poreuse, similaire à celle d'un tissu vivant comme représenté à la Figure 2. L'observation en Cryo-Microscopie Electronique à Balayage de l'hydrogel hydraté, comme représenté à la Figure 1, montre une taille de pores de 1-3 µm n'autorisant pas la pénétration des cellules au sein de l'hydrogel mais autorisant la libre diffusion des nutriments et des déchets cellulaires.

### Exemple 2 : Synthèse de particules/fragments d'hydrogel de chitosane afin d'élaborer la structure 3D (structure M1 et structure M2).

### Structure M1 :

L'hydrogel de chitosane obtenu à l'issue de l'exemple 1 est découpé en petits carrés de 1 mm de côté puis placé dans 10 ml d'eau, de préférence stérile. L'hydrogel est alors broyé à l'aide d'un ultra-turrax, à raison de 6 000 à 17 000 tr/min, pendant 10 secondes à 2-4 reprises. En vue d'obtenir des particules de taille homogène et de diamètre attendu, le broyage est réalisé préférentiellement à 6 000 tr/min pendant 10 secondes à 3 reprises, permettant d'atteindre des tailles de particules de : 400-700 µm (50%), voire de 250-900 µm (> 80%), avec une moyenne de l'ordre de 650 microns.

La solution obtenue est centrifugée, préférentiellement à 1375g pendant 7 minutes, en vue de récupérer le culot constitué de particules d'hydrogel de chitosane. La figure 3 illustre un exemple de particules de chitosane obtenues. Une mini-cuillère est utilisée pour doser la quantité de particules de chitosane qui sera mise en contact avec les cellules chondrogéniques. Des tests préliminaires ont permis de valider la reproductibilité de la mesure.

### Structure M2 :

En vue de renforcer les propriétés visco-élastiques de la structure 3D dans laquelle les chondrocytes sont ensemencées, les inventeurs ont également réalisé une seconde structure (M2), en ajoutant un constituant anionique, interagissant avec les fonctions cationiques du chitosane. Le polymère choisi est l'acide hyaluronique, de préférence d'origine bactérienne car un tel constituant est connu pour ses meilleures propriétés de biocompatibilité, afin d'éviter les allergies ou les rejets éventuels. La masse moléculaire en poids de l'acide hyaluronique utilisé dans la réalisation de la structure M2 est d'environ 2 MDa. L'acide hyaluronique est ajouté après la préparation des particules d'hydrogel de chitosane.

### Exemple 3 : Mise en culture de cellules au sein de la structure 3D.

Les cellules utilisées dans le cadre de cet exemple sont des chondrocytes humains obtenus à partir de prélèvements humains et traités selon le protocole décrit dans le document FR2965278 (Univ. de Caen Basse-Normandie, et al).
Les particules d'hydrogel obtenues à l'issue de l'exemple 2, avec l'acide hyaluronique (structure 3D M2) ou bien sans (structure 3D M1), sont stérilisés, par exemple à 121°C pendant 15 min, avant la mise en contact avec les cellules. On prélève quelques mini-cuillères de particules d'hydrogel, de préférence 2 mini-cuillères correspondant à 80 à 84 particules, qu'on introduit dans le puits d'une plaque de culture 24 puits préalablement recouvert d'un insert (taille pores 8 µm). On y ajoute les cellules, entre 10⁵ et 10⁷, préférentiellement de l'ordre de 6.10⁵ cellules/puits, pour 80-84 particules d'hydrogel, qu'on mélange délicatement avec les particules d'hydrogel de chitosane. Cette proportion de cellules par rapport à la structure 3D correspond à environ 6,7 x 10⁶ cellules par gramme de structure 3D au moment de l'ensemencement.
La culture est réalisée en atmosphère contrôlée dans une étuve à 37°C avec un taux en CO2 de 5%, en conditions de normoxie.
Les cellules adhèrent spontanément aux particules d'hydrogel de chitosane. La quantité de cellules tombant au fond du puits est considérée comme négligeable.
A titre de contrôle, 6.10⁵ cellules, obtenues comme décrit précédemment, sont mises en culture en monocouches sur plastique dans des plaques 24 puits avec des conditions de culture identiques à celles décrites plus haut pour les structures 3D (atmosphère contrôlée dans une étuve à 37°C, avec un taux en CO₂ de 5%, normoxie). Les cellules y adhèrent également spontanément.

### Exemple 4 : Etape de prolifération des cellules.

Dans cette étape, le milieu de culture choisi est favorable à la multiplication des cellules.
Le milieu choisi est une solution de 50/50 DMEM-HAM F12 + 1% AB (streptomycine/pénicilline) + 10 SVF additionnée d'une solution dite « FI » comprenant FGF-2 à 5ng/ml + Insuline à 5µg/ml. (Claus et al ; 2012).
Le mélange décrit à l'étape précédente est recouvert, après un court temps, de ce milieu de culture connu pour être favorable à la prolifération des cellules.
Durant cette phase d'amplification, le milieu de culture est renouvelé 3 fois par semaine, pour les cultures dans les structures 3D comme pour les cultures en monocouches. La période de prolifération dure entre une et deux semaines pour l'obtention d'un nombre suffisant de cellules. Les inventeurs ont observé que la phase d'amplification durait environ deux semaines, quand les cellules étaient ensemencées dans une structure constituée de particules d'hydrogel pur (structure 3D M1) et pouvait être écourtée à 1 semaine en présence de particules d'hydrogel supplémentées par des chaines linéaires d'une molécule anionique telle que l'acide hyaluronique (structure 3D M2).
Par ailleurs, la quantité initiale de cellules de 6.10⁵ cellules/insert peut tout à fait être augmentée, si la condition du nombre de cellules/masse d'hydrogel ou nombre de cellules/volume d'hydrogel, ou nombre de cellules/nombre de particules d'hydrogel est respectée. A titre d'exemple, il est tout à fait envisageable d'ensemencer 1 à 1,5 x 10⁶ cellules/insert, à condition d'y ajouter la quantité nécessaire en structure 3D, en vue d'obtenir plus de 4 x10⁶ cellules/insert en fin de procédé, voire même 10,5 x 10⁶ cellules/insert, voire plus.

### Suivi par microscopie optique :

Un suivi par microscopie optique en phase inversé a été réalisé. Il a permis de valider que les cellules adhèrent bien aux particules d'hydrogel de chitosane et que cet environnement est favorable à leur culture. Les conditions de culture (structure tridimensionnelle M1 ou M2, et milieu de culture FI) favorisent la prolifération et la division des chondrocytes.
Les cellules observées peuvent proliférer soit de façon isolées soit en amas/grappe. Les cultures en structures 3D de particules d'hydrogel montrent des cellules majoritairement rondes. La forme allongée, caractéristique des fibroblastes, n'est pas observée dans les structures 3D M1 et M2, sauf parfois à la périphérie, c'est-à-dire à l'interface entre la structure et le milieu extérieur.

A titre de contrôle, les inventeurs ont réalisé en parallèle des cultures en monocouches. Au bout de 24 heures de culture, dans le même milieu FI que les cellules ensemencées dans les structures M1 et M2, les chondrocytes adoptent une morphologie allongée caractéristique des cellules fibroblastiques.

### Viabilité des cellules :

La viabilité des chondrocytes ensemencés dans les structures tridimensionnelles a été mesurée avec le kit Live and Dead sur des fractions de cultures à 7 jours (en cours d'amplification, en milieu FI). Le dénombrement des cellules mortes (rouges) et des cellules vivantes (vert) est réalisé avec le logiciel ImageJ à partir de clichés pris en microscopie à fluorescence au x20. Le pourcentage de cellules mortes est calculé pour chaque condition en faisant le rapport cellules mortes / cellules totales. La viabilité est supérieure à 93%, voire supérieure à 97% ce qui montre une bonne biocompatibilité de la structure 3D.
La figure 4 illustre les résultats obtenus.

### Tests de prolifération :

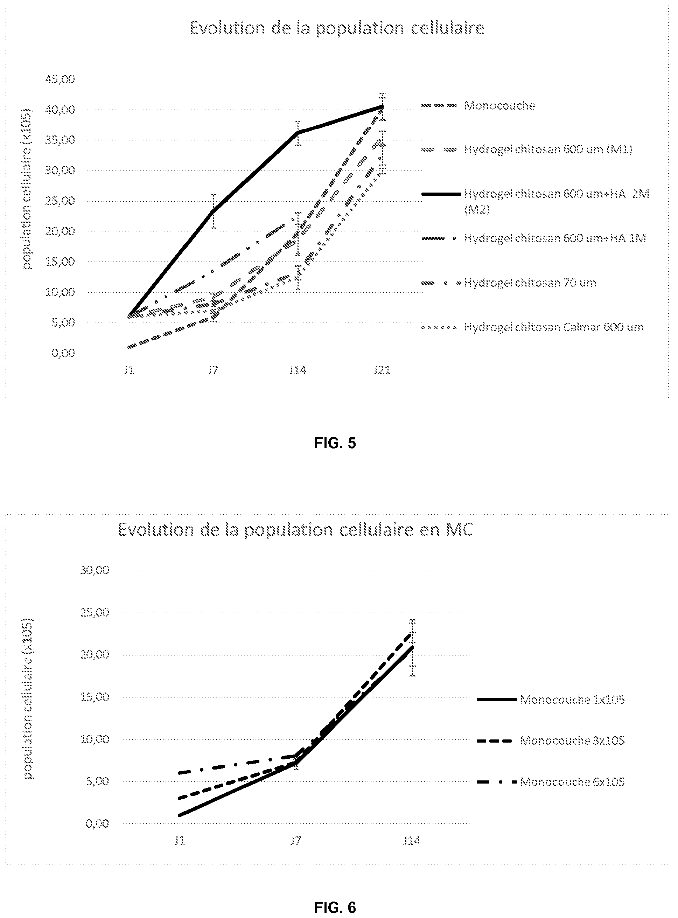

Des tests de prolifération ont été réalisés par le comptage des cellules totales au Cellometer T4 après décollement des cellules à la trypsine et coloration des cellules mortes au bleu trypan.
Les mesures ont été réalisées après 1 jour (J1), 14 jours (J14) et 21 jours (J21) de culture après ensemencement de chondrocytes primaires à J0.
La figure Fig.5 illustre l'évolution de la population cellulaire.
Après 7 jours, l'augmentation du nombre de cellules est clairement observée. Les cellules survivent et prolifèrent très bien en structure 3D d'hydrogel comme en monocouches (MC).
En structure tridimensionnelle, M1 ou M2, les cellules restent rondes pendant l'étape de multiplication alors qu'elles adoptent une forme allongée, de type fibroblastique, en monocouches.
Par ailleurs, la figure 6 illustre qu'en monocouches, la population cellulaire est identique à partir de 7 jours, et ce quelle que soit la densité initiale de cellules ensemencées.

En conclusion, à l'issue de cette étape de prolifération, il est observé que l'amplification des cellules en structure tridimensionnelle composée de particules d'hydrogel de chitosane pur (structure M1) est quasiment aussi productive qu'en monocouches (MC), qui est le protocole de référence pour la multiplication des cellules telles que les chondrocytes, mais qui impose une étape de trypsinisation qui peut être évitée en utilisant la structure M1.
L'addition d'acide hyaluronique à la structure tridimensionnelle de particules d'hydrogel de chitosane (structure M2) entraine une très forte accélération de la prolifération des cellules, bien supérieure à la structure M1 ou à la culture en monocouches, notamment d'un facteur 2.

### Exemple 5 : Différenciation et production de matrice extracellulaire

Dans le cadre de la présente invention, les étapes de multiplication et de différenciation sont préférentiellement distinctes : dans un premier temps les cellules se multiplient et dans un second temps, elles se différencient et produisent de la matrice extracellulaire. Le milieu de culture utilisé pour l'étape précédente de multiplication est modifié au bout de 15 jours. On remplace le milieu de culture « FI » par un milieu « BIT » en vue de favoriser l'étape de différenciation des cellules et de production de matrice extra-cellulaire.

Le milieu de culture est alors préférentiellement composé de : 50/50 DMEM-HAM F12+1% AB (streptomycine/pénicilline)+ 10 SVF, auquel est additionné une solution BIT composée de : BMP-2 à 200ng/ml + Insuline à 5µg/ml + triiodothyronine T3 à 100mM (Claus et al, 2012).

Ce nouveau milieu de culture est renouvelé tous les deux ou trois jours. La période de re-différenciation et de chondrogenèse dure préférentiellement 3 semaines. A titre de contrôle, on opère le même changement de milieu pour les cellules cultivées en monocouches.

### Suivi par microscopie optique :

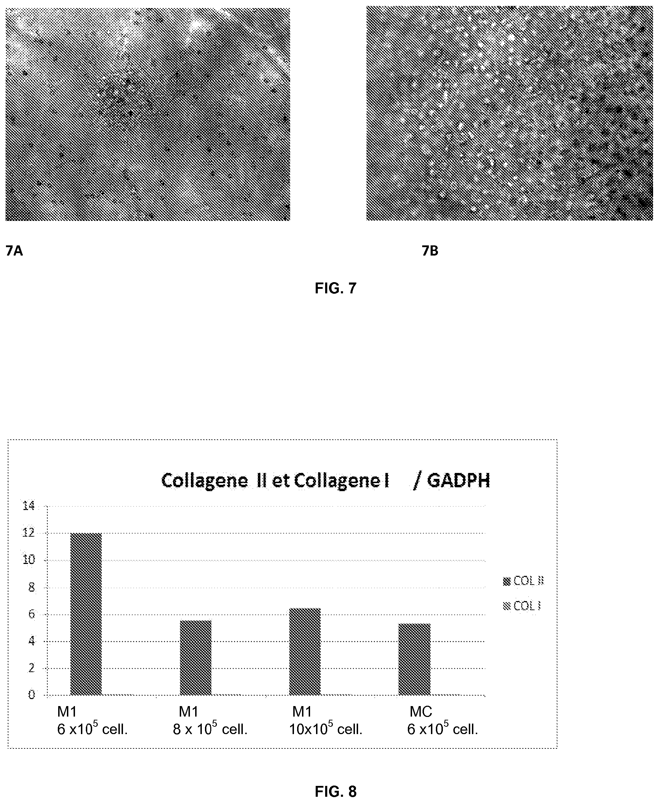

Il est connu que les cellules d'aspect fibrillaire en monocouches en milieu FI s'arrondissent après 1 semaine de culture en milieu BIT. Les cellules cultivées en monocouches (correspondant au contrôle) changent d'aspect après le passage en milieu BIT, ce qui indique que le changement de milieu de culture induit bien un changement de comportement des cellules.
Dans les hydrogels (structures M1 et M2), les cellules sont majoritairement rondes à l'issue de la phase d'amplification et continuent à l'être durant toute la phase de production de matrice extracellulaire. Ce point est illustré notamment à la figure Fig.7.
En fin de culture, à J35, les cellules sont parfaitement rondes en hydrogels (structures M1 et M2), moins en monocouches.
En structures tridimensionnelles, les cellules peuvent agglutiner les particules d'hydrogel et former une sorte de « bille » plus ou moins compacte. Cette observation est réalisée dans la plupart des conditions contenant les hydrogels, pas dans les monocouches toutefois qui servent de contrôle. Cette observation constitue une preuve de la forte production de matrice extracellulaire qui s'est accumulée autour des cellules. Les cellules fabriquent plus de matrice extracellulaire en environnement tridimensionnel qu'en monocouches.
Il est à noter que la composition constituée desdites billes, demeure toutefois, sous certaines conditions, injectable, malgré la synthèse d'une quantité importante de matrice extracellulaire. Quoi qu'il en soit, la composition est implantable.

### Tests PCR :

Par PCR, il a été quantifié le taux de transcription des protéines suivantes : COLI, COLII et GAPDH. Le taux de transcription sert de référence pour comparer les niveaux de transcription COLI et COLII. En effet, il est bien connu que le phénotype chondrocytaire et la production de matrice extracellulaire s'accompagne d'une forte synthèse de transcrits COLII, alors que les transcrits COLI accompagnent généralement le processus de dé-différenciation, notamment vers un phénotype fibroblastique. Les résultats sont présentés à la figure 8.

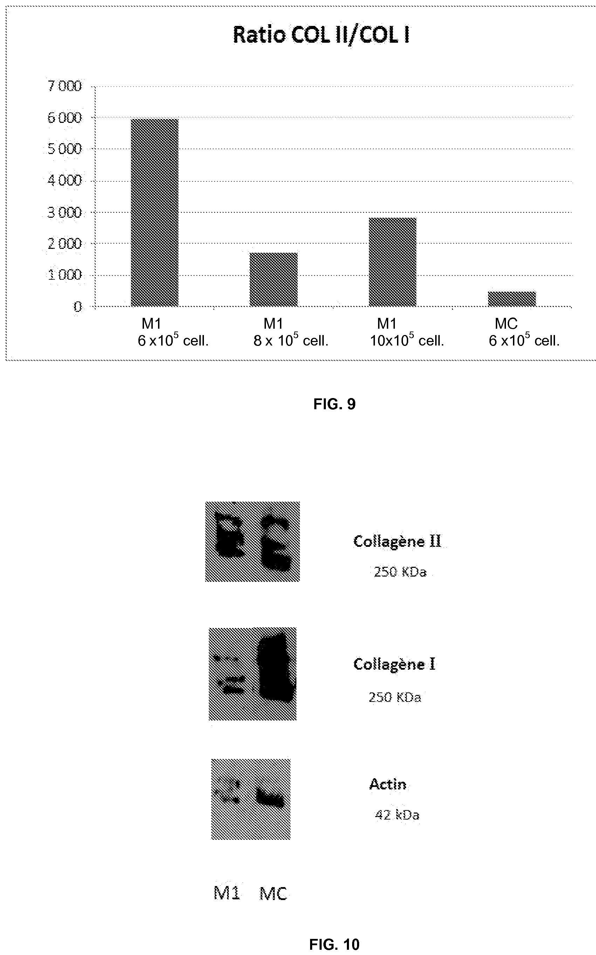

Les résultats COLII/GAPDH réalisés à l'issue de l'étape de synthèse de MEC, montrent qu'il y a plus de transcrits de COLII pour les cultures en hydrogel que pour les cultures en monocouches. Les résultats COLI/GAPDH montrent qu'il y a, au contraire, plus de transcrits de COLI dans les cultures en monocouches que dans les cultures au sein des structures tridimensionnelles.
Le résultat du calcul du ratio COLII/COLI est illustré à la figure Fig.9. Il montre qu'il y a un bien meilleur ratio, et donc une bien meilleure re-différenciation après dé-différenciation, au sein des structures 3D constituées de particules d'hydrogel de chitosane comparé aux monocouches. L'environnement tridimensionnel testé favorise donc bien la re-différenciation de chondrocytes dé-différenciés, suite à une intense multiplication préalable, d'un facteur au moins 6. Cette structure 3D favorise l'expression du phénotype chondrocytaire.

Les conditions de culture (structure tridimensionnelle à base d'hydrogel de chitosane, et milieu de culture BIT) favorisent donc la re-différenciation des chondrocytes et la production de matrice cartilagineuse, comparativement à une culture en monocouches.

### Tests en Western Blot :

Après avoir vérifié les taux de transcription des gènes ColI et ColII, en fonction des conditions de culture (3D ou monocouches), il a été vérifié le taux de synthèse des protéines correspondantes, par la technique du Western Blot. Les résultats des différents Western Blots sont illustrés à la Fig.10.
Les résultats du Western Blot anti-COLII montrent la présence de COLII dans toutes les conditions. Les résultats des WB anti-COLI, montrent des spots plus intenses en monocouches. Cette observation corrobore ce qui a été révélé en Q-PCR : le ratio COLII/COLI est supérieur dans les structures 3D d'hydrogel qu'en monocouches.
L'analyse Western Blot montre l'expression des protéines caractéristiques du cartilage articulaire dans la structure 3D.

### Résultats d'immunohistochimie :

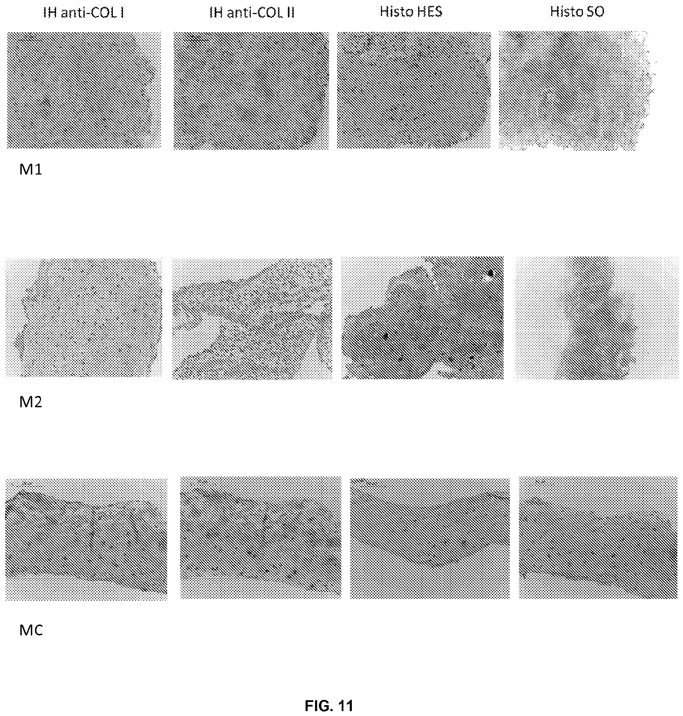

Les compositions obtenues ont ensuite été observées en immunohistochimie, afin de comparer la mise en œuvre du nouveau procédé, en structure tridimensionnelle, et la culture traditionnelle en monocouches, au niveau de la synthèse de MEC, de protéoglycannes, de collagène de types I et II. Les résultats sont illustrés par les photos de la figure Fig.11.
On observe bien la présence plus importante de protéoglycannes dans les structures tridimensionnelles (M1 et M2 à la figure 11) qu'en monocouches (MC à la figure 11) grâce à la coloration à la Safranine O (SO), qui met en évidence la présence de GAG. Par ailleurs, on observe la production importante de matrice extra-cellulaire et de collagène de type II sur les clichés d'immunohistochimie des cellules en environnement tridimensionnel, marquées respectivement par la coloration HES, qui met en évidence les noyaux et la MEC, et par l'immunomarquage de Collagène II, qui met en évidence la présence de COLII. La coloration des cellules au sein de la matrice par immuno-marquage Collagène I confirme par ailleurs la quasi absence de collagène I, lorsque les chondrocytes sont cultivées au sein de structures tridimensionnelles.

### Exemple 6 : Greffe de chondrocytes

L'ensemble cellules et structure 3D (soit la structure M1 constituée de particules d'hydrogel de chitosane, ou bien la structure M2, constituée de particules d'hydrogel de chitosane supplémentées avec une molécule anionique de type acide hyaluronique) constitue en fin de culture, soit entre 3 et 6 semaines, un néo-tissu cartilagineux qui peut être injecté ou bien implanté par arthroscopie.
Il est évidemment possible d'augmenter le nombre de cellules dans un insert en augmentant la quantité d'hydrogel, en respectant toutefois les mêmes conditions de nombre de cellules par rapport à la masse d'hydrogel, ou au nombre de particules d'hydrogel, constituant la structure 3D.
A titre d'exemple, pour un prélèvement de 0,3g-0,5g de cartilage humain, il peut être extrait 1-1,5x10⁶ cellules (chondrocytes). Dans la mesure où les inventeurs ont montré, dans les précédents exemples, qu'à partir de 6.10⁵ cellules initiales par insert, il est possible d'en obtenir 3,6x10⁶ cellules/insert dans 0,09 g de structure hydrogel, correspondant à 80-84 particules d'hydrogel, on obtient les données de concentration suivantes :
- Concentration initiale de 6,7x10⁶ cellules/g de biomatériau,
- Concentration finale supérieure à 40 x10⁶ cellules/g de biomatériau (structure 3D).
Pour un prélèvement de 1 à 1,5.10⁶ cellules, on peut donc atteindre plus de.3.10⁶ cellules, voire même plus de 10,5.10⁶ cellules, en fin de procédé, en 2-5 semaines, ce qui est amplement suffisant en vue d'une greffe où les quantités recommandées sont de 3,2-6,5 x10⁶ cellules.

En conclusion des précédents exemples, il est observé les points suivants :
Durant la phase d'amplification/multiplication :
- un rendement équivalent voire supérieur en structure tridimensionnelle comprenant des particules d'hydrogel de chitosane pur comparativement à la culture en monocouches,
- un rendement bien supérieur en structure tridimensionnelle comprenant des particules d'hydrogel supplémentées par de l'acide hyaluronique qu'en monocouches.

Durant la phase de différenciation et de production de MEC :
- une re-différenciation des cellules en structures 3D et en monocouches comme prouvé par les analyses PCR, WB et immunohistochimie;
- un ratio des ARN messagers COLII/COLI significativement supérieur en structure tridimensionnelle comparativement à la culture en monocouches,
- un rapport protéique COLII/COLI significativement supérieur en structure tridimensionnelle qu'en monocouches
- un phénotype chondrocytaire stable en structure 3D
- la production de matrice cartilagineuse abondante en structure 3D

La succession des étapes, dans un même milieu tridimensionnel, comprenant des particules d'hydrogel de chitosane avec/sans molécule structurante, d'amplification puis de différenciation/chondrogénèse est très favorable à l'obtention d'un néo-tissu cartilagineux injectable ou implantable présentant d'excellentes propriétés mécaniques et biologiques. L'addition d'acide hyaluronique améliore le système en accélérant le processus d'amplification des cellules et permettant d'augmenter le nombre de cellules à implanter ou de gagner une semaine sur le protocole global.
La configuration de la structure permet d'optimiser la surface de contact avec les cellules.

### Exemple 7 : Comparaison des différentes structures 3D.

Les inventeurs ont reproduit les structures 3D décrites dans les exemples précédents, notamment à l'exemple 2, en variant le type de chitosane constituant les particules d'hydrogel, la taille des particules d'hydrogel, et la présence et la concentration d'acide hyaluronique. Les taux de prolifération ont été comparés et les résultats obtenus sont illustrés dans le tableau 1. La valeur 1 a été attribuée à la structure M1 correspondant à des particules de plusieurs centaines de microns, obtenus à partir de chitosane de champignons.
Le taux de prolifération obtenu avec la structure M2 (extrait de champignons et supplémenté avec l'acide hyaluronique à 2M) est 2 fois supérieur par rapport à la structure M1 (extrait de champignons non supplémenté avec l'acide hyaluronique), qui elle-même permet d'obtenir un taux de prolifération 1,5 fois supérieur par rapport à du chitosane extrait de calmars, ou bien avec des particules de taille de l'ordre de dizaines de µm.

**Tableau 1 : Ratios de prolifération.**

| | Fragments de centaines de µm | Fragments de dizaines de µm |
|---|---|---|
| Chitosane extrait de champignons supplémenté avec HA 2M | 2** | |
| Chitosane extrait de champignons supplémenté avec HA 1M | 1,2 | |
| Chitosane extrait de champignons | 1* | 0,67 |
| Chitosane extrait de calmars | 0,67 | |

| | | |
|---|---|---|
| *structure M1 ; ** structure M2 | | |

### REFERENCES:

Claus S, Mayer N, Aubert-Foucher E, Chajra H, Perrier-Groult E, Lafont J, Piperno M, Damour O, Mallein-Gerin F. Cartilage-characteristic matrix reconstruction by sequential addition of soluble factors during expansion of human articular chondrocytes and their cultivation in collagen sponges. Tissue Eng Part C Methods. 2012;18(2):104-12.
Correia CR, et al. Chitosan scaffolds containing hyaluronic acid for cartilage tissue engineering. Tissue Eng Part C Methods. 2011 Jul;17(7):717-30.
Denuziere A, Ferrier D, Damour O, Domard A. Chitosan-chondroitin sulfate and chitosan-hyaluronate polyelectrolyte complexes: biological properties. Biomaterials. 1998; 19(14): 1275-85. Griffon DJ, Sedighi MR, Schaeffer DV, Eurell JA, Johnson AL. Chitosan scaffolds: interconnective pore size and cartilage engineering. Acta Biomater. 2006 May;2(3):313-20. Hao T, Wen N, Cao JK, Wang HB, Lü SH, Liu T, Lin QX, Duan CM, Wang CY. The support of matrix accumulation and the promotion of sheep articular cartilage defects repair in vivo by chitosan hydrogels. Osteoarthritis Cartilage. 2010 Feb;18(2):257-65.
Hautier A, et al. Bone morphogenetic protein-2 stimulates chondrogenic expression in human nasal chondrocytes expanded in vitro. Growth Factors. 2008;26(4):201-11.
Hoemann CD, Sun J, Légaré A, McKee MD, Buschmann MD. Tissue engineering of cartilage using an injectable and adhesive chitosan-based cell-delivery vehicle. Osteoarthritis Cartilage. 2005; 13(4):318-29.
Lahiji A, Sohrabi A, Hungerford DS, Frondoza CG. Chitosan supports the expression of extracellular matrix proteins in human osteoblasts and chondrocytes. J Biomed Mater Res. 2000;51(4):586-95.
Liu G, et al. Optimal combination of soluble factors for tissue engineering of permanent cartilage from cultured human chondrocytes. J Biol Chem. 2007 Jul 13;282(28):20407-15.
Montembault A, Tahiri K, Korwin-Zmijowska C, Chevalier X, Corvol MT, Domard A. A material decoy of biological media based on chitosan physical hydrogels: application to cartilage tissue engineering. Biochimie. 2006 May;88(5):551-64.
Park H, Choi B, Hu J, Lee M. Injectable chitosan hyaluronic acid hydrogels for cartilage tissue engineering. Acta Biomater. 2013 Jan;9(1):4779-86.
Suh JK, Matthew HW. Application of chitosan-based polysaccharide biomaterials in cartilage tissue engineering: a review. Biomaterials. 2000;21(24):2589-98.
Tallheden T, et al. Proliferation and differentiation potential of chondrocytes from osteoarthritic patients. Arthritis Res Ther. 2005;7(3):R560-8.

## Revendications

1. Procédé *in vitro* d'obtention d'une composition implantable pour la réparation tissulaire du cartilage comprenant des particules d'hydrogel de chitosane et des cellules aptes à former du cartilage hyalin, ledit procédé comprenant les étapes successives suivantes :
(i.) Amplification de cellules primaires, dans une structure tridimensionnelle comprenant des particules d'hydrogel physique de chitosane ou de dérivé de chitosane, puis
(ii.) Induction de la synthèse de matrice extracellulaire par lesdites cellules amplifiées, au sein de la structure tridimensionnelle de l'étape (i.),
où lesdites cellules sont des chondrocytes articulaires primaires et/ou des cellules souches mésenchymateuses primaires différenciées en chondrocytes et lesdites cellules ne sont pas des cellules souches embryonnaires humaines,
et où lesdites cellules ne pénètrent pas dans les particules d'hydrogel.

2. Procédé selon la revendication 1, où les deux étapes (i) et (ii) se déroulent au sein de la même structure tridimensionnelle sans aucune étape de trypsinisation des cellules.

3. Procédé selon l'une quelconque des revendications 1 à 2, où ladite composition est réalisée en moins de 45 jours, de préférence moins de 35 jours, notamment moins de 28 jours.

4. Procédé selon l'une quelconque des revendications précédentes, où l'étape d'amplification dure entre 1 à 3 semaines, de préférence moins de deux semaines, et permet la multiplication du nombre de cellules par au moins 4, de préférence par 6.

5. Procédé selon l'une quelconque des revendications 1 à 4 où les particules d'hydrogel sont de taille moyenne comprise entre 10 µm et 1,5 mm, de préférence entre 400 µm et 700 µm.

6. Procédé selon l'une quelconque des revendications 1 à 5 où le chitosane a une masse molaire moyenne en poids supérieure à 50 kDa, de préférence entre 150 et 220 kDa.

7. Procédé selon l'une quelconque des revendications 1 à 6 où le chitosane est extrait à partir de champignons, de préférence à partir d'*Agaricus bisporus.*

8. Procédé selon l'une quelconque des revendications 1 à 7 où la structure tridimensionnelle comprend également un polymère anionique associé aux particules d'hydrogel de chitosane en surface, de préférence de l'acide hyaluronique, qui est présent durant les deux étapes ou bien ajouté à l'issue de l'étape (i.) ou (ii.).

9. Procédé selon l'une quelconque des revendications 1 à 8 où l'acide hyaluronique est extrait à partir de fermentation bactérienne, avec une masse molaire moyenne en poids supérieure à 1MDa, de préférence supérieure à 2 MDa.

10. Procédé selon l'une quelconque des revendications 1 à 9, où le taux de viabilité des cellules est supérieur à 90%, de préférence supérieur à 93%.

11. Composition implantable comprenant une structure tridimensionnelle formée de particules d'hydrogel physique de chitosane ou de dérivé de chitosane, associées à un polymère anionique, de préférence l'acide hyaluronique, et des chondrocytes différenciés ou toutes cellules différenciées en chondrocytes, ou des cellules souches différenciées en chondrocytes, ou des cellules précurseurs de chondrocytes, ou des cellules pluripotentes induites (IPS), ou des cellules souches mésenchymateuses, où lesdites cellules ne sont pas des cellules souches embryonnaires humaines,
et où ladite composition est susceptible d'être obtenue par le procédé selon l'une quelconque des revendications 1 à 10.

12. Composition implantable selon la revendication 11, où les chondrocytes synthétisent préférentiellement des protéines de collagène de type II et des ARN messagers de collagène de type II, exprimant un rapport COLII/COLI supérieur à 1.

13. Composition implantable par arthroscopie selon l'une quelconque des revendications 11 à 12, pour une utilisation dans la réparation cartilagineuse ou pour une utilisation comme implant, ou comme greffe ou comme vecteur de principe actif chez l'homme ou chez l'animal.

14. Composition implantable selon l'une quelconque des revendications 11 à 13, où la teneur en eau au sein de l'hydrogel de chitosane est supérieure à 70%, de préférence supérieure à 80%.

15. Composition implantable selon l'une quelconque des revendications 11 à 14, où l'hydrogel physique de chitosane est synthétisé sans agent de réticulation.

16. Structure tridimensionnelle formée de particules d'hydrogel physique de chitosane ou de dérivé de chitosane, associées à un polymère d'acide hyaluronique, où ledit chitosane est extrait à partir de champignons, et où lesdites particules d'hydrogel sont de taille moyenne comprise entre 400 µm et 700 µm.

17. Structure tridimensionnelle selon la revendication 16, assurant une distribution spatiale optimale des cellules ensemencées.

18. Utilisation d'une structure tridimensionnelle formée de particules d'hydrogel physique de chitosane ou de dérivé de chitosane, associés à un polymère anionique, de préférence de l'acide hyaluronique, pour l'ensemencement de chondrocytes, ou de cellules souches différenciées en chondrocytes, ou de cellules précurseurs de chondrocytes, ou de cellules pluripotentes induites (IPS), ou de cellules souches mésenchymateuses aux fins de prolifération et de synthèse de matrice extracellulaire desdites cellules, où lesdites cellules ne sont pas des cellules souches embryonnaires humaines.

19. Utilisation d'une structure tridimensionnelle selon la revendication 16 ou 17, pour l'ensemencement de cellules, de préférence de cellules osseuses, de fibroblastes, de kératinocytes ou des combinaisons de certaines de ces cellules.

## Patentansprüche

1. *In-vitro-* Verfahren zum Erhalten einer implantierbaren Zusammensetzung zum Reparieren des Knorpelgewebes, umfassend Chitosanhydrogelpartikel und Zellen, die in der Lage sind, hyalinen Knorpel zu bilden, wobei das Verfahren die folgenden aufeinanderfolgenden Schritte umfasst:
(i.) Amplifizieren von Primärzellen in einer dreidimensionalen Struktur, die Partikel eines physikalischen Chitosanhydrogels oder eines Chitosanderivats umfasst, dann
(ii.) Induzieren der extrazellulären Matrixsynthese durch die amplifizierten Zellen innerhalb der dreidimensionalen Struktur von Schritt (i.),
wobei die Zellen primäre artikuläre Chondrozyten und/oder primäre mesenchymale, in Chondrozyten differenzierte Stammzellen sind, und die Zellen keine menschlichen embryonalen Stammzellen sind,
und wobei die Zellen die Hydrogelpartikel nicht durchdringen.

2. Verfahren nach Anspruch 1, wobei die zwei Schritte (i) und (ii) innerhalb derselben dreidimensionalen Struktur ohne irgendeinen Schritt der Trypsinisierung der Zellen stattfinden.

3. Verfahren nach einem der Ansprüche 1 bis 2, wobei die Zusammensetzung in weniger als 45 Tagen, vorzugsweise weniger als 35 Tagen, insbesondere weniger als 28 Tagen, hergestellt wird.

4. Verfahren nach einem der vorhergehenden Ansprüche, wobei der Amplifizierungsschritt zwischen 1 und 3 Wochen dauert, vorzugsweise weniger als zwei Wochen, und die Multiplikation der Anzahl von Zellen mit mindestens 4, vorzugsweise mit 6, ermöglicht.

5. Verfahren nach einem der Ansprüche 1 bis 4, wobei die Hydrogelpartikel eine durchschnittliche Größe zwischen 10 µm und 1,5 mm, vorzugsweise zwischen 400 µm und 700 µm, aufweisen.

6. Verfahren nach einem der Ansprüche 1 bis 5, wobei das Chitosan eine gewichtsmittlere molare Masse von über 50 kDa, vorzugsweise zwischen 150 und 220 kDa, aufweist.

7. Verfahren nach einem der Ansprüche 1 bis 6, wobei das Chitosan aus Pilzen, vorzugsweise aus *Agaricus bisporus,* extrahiert wird.

8. Verfahren nach einem der Ansprüche 1 bis 7, wobei die dreidimensionale Struktur ebenfalls ein anionisches Polymer umfasst, das mit den Chitosanhydrogelpartikeln auf der Oberfläche verbunden ist, vorzugsweise Hyaluronsäure, die während der zwei Schritten vorhanden ist oder nach Abschluss von Schritt (i.) oder (ii.) hinzugefügt wird.

9. Verfahren nach einem der Ansprüche 1 bis 8, wobei die Hyaluronsäure aus einer bakteriellen Fermentation mit einer gewichtsmittleren molaren Masse von über 1 MDa, vorzugsweise über 2 MDa, extrahiert wird.

10. Verfahren nach einem der Ansprüche 1 bis 9, wobei die Lebensfähigkeitsrate der Zellen über 90 %, vorzugsweise über 93 %, beträgt.

11. Implantierbare Zusammensetzung, umfassend eine dreidimensionale Struktur, die aus Partikeln eines physikalischen Chitosanhydrogels oder eines Chitosanderivats, die mit einem anionischen Polymer verbunden sind, vorzugsweise Hyaluronsäure, und differenzierten Chondrozyten oder beliebigen Zellen, die in Chondrozyten differenziert sind, oder Stammzellen, die in Chondrozyten differenziert sind, oder Chondrozyten-Vorläuferzellen oder induzierten pluripotenten Zellen (IPS) oder mesenchymalen Stammzellen gebildet ist, wobei die Zellen keine menschlichen embryonalen Stammzellen sind
und wobei die Zusammensetzung durch das Verfahren nach einem der Ansprüche 1 bis 10 erhaltbar ist.

12. Implantierbare Zusammensetzung nach Anspruch 11, wobei die Chondrozyten vorzugsweise Kollagenproteine des Typs II und Kollagen-mRNAs vom TypII synthetisieren, die ein Verhältnis COLII/COLI über 1 exprimieren.

13. Arthroskopisch implantierbare Zusammensetzung nach einem der Ansprüche 11 bis 12 zur Verwendung bei der Knorpelreparatur oder zur Verwendung als Implantat oder als Transplantat oder als Wirkstoffvektor bei Menschen oder Tieren.

14. Implantierbare Zusammensetzung nach einem der Ansprüche 11 bis 13, wobei der Wassergehalt innerhalb des Chitosanhydrogels über 70 %, vorzugsweise über 80 %, beträgt.

15. Implantierbare Zusammensetzung nach einem der Ansprüche 11 bis 14, wobei das physikalische Chitosanhydrogel ohne Vernetzungsmittel synthetisiert wird.

16. Dreidimensionale Struktur, die aus Partikeln eines physikalischen Chitosanhydrogels oder eines Chitosanderivats gebildet ist, die mit einem Hyaluronsäurepolymer verbunden sind, wobei das Chitosan aus Pilzen extrahiert ist und wobei die Hydrogel-Partikel eine mittlere Größe zwischen 400 µm und 700 µm aufweisen.

17. Dreidimensionale Struktur nach Anspruch 16, die eine optimale räumliche Verteilung der ausgesäten Zellen gewährleistet.

18. Verwendung einer dreidimensionalen Struktur aus Partikeln eines physikalischen Chitosanhydrogels oder Chitosanderivats, die mit einem anionischen Polymer verbunden sind, vorzugsweise Hyaluronsäure, zum Animpfen von Chondrozyten oder von in Chondrozyten differenzierten Stammzellen oder von Vorläuferzellen von Chondrozyten oder von induzierten pluripotenten Zellen (IPS) oder von mesenchymalen Stammzellen zum Zweck der Proliferation und Synthese von extrazellulärer Matrix der Zellen, wobei die Zellen keine menschlichen embryonalen Stammzellen sind.

19. Verwendung einer dreidimensionalen Struktur nach Anspruch 16 oder 17 zum Animpfen von Zellen, vorzugsweise von Knochenzellen, von Fibroblasten, von Keratinozyten oder von Kombinationen einiger dieser Zellen.

## Claims

1. An *in vitro* method for obtaining an implantable composition for cartilage tissue repair comprising particles of chitosan hydrogel and cells that are capable of forming hyaline cartilage, said method comprising the following successive steps:
(i) amplification of primary cells in a three-dimensional structure comprising particles of physical hydrogel of chitosan or chitosan derivative, then
(ii) induction of the synthesis of extracellular matrix by said amplified cells within the three-dimensional structure of step (i),
in which said cells are primary articular chondrocytes and/or primary mesenchymal stem cells differentiated into chondrocytes and said cells are not human embryo stem cells, and in which said cells do not penetrate into the hydrogel particles.

2. The method according to claim 1, wherein the two steps (i) and (ii) are carried out within the same three-dimensional structure without any cell trypsinization step.

3. The method according to any one of claims 1 to 2, wherein said composition is produced in less than 45 days, preferably less than 35 days, in particular less than 28 days.

4. The method according to any one of the preceding claims, wherein the duration of the amplification step is between 1 and 3 weeks, preferably less than two weeks, and enables the number of cells to be multiplied by at least 4, preferably by 6.

5. The method according to any one of claims 1 to 4, wherein the hydrogel particles have a mean size in the range 10 µm to 1.5 mm, preferably in the range 400 µm to 700 µm.

6. The method according to any one of claims 1 to 5, wherein the chitosan has a weight average molecular weight higher than 50 kDa, preferably between 150 and 220 kDa.

7. The method according to any one of claims 1 to 6, wherein the chitosan is extracted from fungi, preferably from *Agaricus bisporus.*

8. The method according to any one of claims 1 to 7, wherein the three-dimensional structure also comprises an anionic polymer associated with particles of chitosan hydrogel at the surface, preferably hyaluronic acid, which is present during the two steps or is added at the end of step (i) or (ii).

9. The method according to any one of claims 1 to 8, wherein the hyaluronic acid is extracted by bacterial fermentation, with a molecular mass by weight of more than 1 MDa, preferably more than 2 MDa.

10. The method according to any one of claims 1 to 9, wherein the cell viability is more than 90%, preferably more than 93%.

11. An implantable composition comprising a three-dimensional structure formed from particles of physical hydrogel of chitosan or chitosan derivative, associated with an anionic polymer, preferably hyaluronic acid, and differentiated chondrocytes or any cells differentiated into chondrocytes, or stem cells differentiated into chondrocytes or precursor cells of chondrocytes, induced pluripotent cells (IPS) or mesenchymal stem cells,
wherein said cells are not human embryo stem cells; and
wherein said composition is obtainable by the method according to any one of claims 1 to 10.

12. The implantable composition according to claim 11, wherein the chondrocytes preferentially synthesize type II collagen proteins and messenger RNA of type II collagen, expressing a COLII/COLI ratio higher than 1.

13. The composition implantable by arthroscopy according to any one of claims 11 to 12, for use in cartilage repair, or for use as an implant, as a graft or as a vector of active principle for humans or animals.

14. The implantable composition according to any one of claims 11 to 13, wherein the water content in the chitosan hydrogel is more than 70%, preferably more than 80%.

15. The implantable composition according to any one of claims 11 to 14, wherein the physical hydrogel of chitosan is synthesized without a cross-linking agent.

16. A three-dimensional structure formed by particles of physical hydrogel of chitosan or of chitosan derivative, associated with a hyaluronic acid polymer, wherein said chitosan is extracted from fungi, and wherein said hydrogel particles have a mean size in the range 400 µm to 700 µm.

17. A three-dimensional structure according to claim 16, ensuring an optimal spatial distribution of the seeded cells.

18. Use of a three-dimensional structure formed by particles of physical hydrogel of chitosan or of chitosan derivative, associated with an anionic polymer, preferably hyaluronic acid, to seed chondrocytes, or stem cells differentiated into chondrocytes or precursor cells of chondrocytes, induced pluripotent cells (IPS) or mesenchymal stem cells, for the purposes of proliferation and synthesis of extracellular matrix by said cells, wherein said cells are not human embryo stem cells.

19. Use of a three-dimensional structure according to claim 16 or 17, for seeding cells, preferably bone cells, fibroblasts, keratinocytes, or combinations thereof.
